(19) Europäisches Patentamt
European Patent Office
Office européen des brevets

(11) EP 0 668 869 B1

(12) EUROPÄISCHE PATENTSCHRIFT

(45) Veröffentlichungstag und Bekanntmachung des Hinweises auf die Patenterteilung:
**13.05.1998 Patentblatt 1998/20**

(21) Anmeldenummer: **92913125.8**

(22) Anmeldetag: **23.06.1992**

(51) Int Cl.[6]: **C07K 5/06**, A61K 38/05

(86) Internationale Anmeldenummer:
**PCT/EP92/01411**

(87) Internationale Veröffentlichungsnummer:
**WO 93/01208 (21.01.1993 Gazette 1993/03)**

(54) **2- 3-(4-AMIDINO-PHENYL)]-PROPIONSÄUREDERIVATE, IHRE HERSTELLUNG UND VERWENDUNG**

2- 3-(4-AMIDINOPHENYL)]PROPIONIC ACID DERIVATIVES, THEIR PREPARATION AND THEIR USE

DERIVES DE L'ACIDE 2- 3-(4-AMIDINO-PHENYL)]-PROPIONIQUE, LEUR FABRICATION ET LEUR UTILISATION

(84) Benannte Vertragsstaaten:
**AT BE CH DE DK ES FR GB IT LI NL SE**

(30) Priorität: **03.07.1991 DE 4121947**

(43) Veröffentlichungstag der Anmeldung:
**30.08.1995 Patentblatt 1995/35**

(73) Patentinhaber: **BASF Aktiengesellschaft**
**67063 Ludwigshafen (DE)**

(72) Erfinder:
- **MACK, Helmut**
  **D-6701 Meckenheim (DE)**
- **PFEIFFER, Thomas**
  **D-6703 Limburgerhof (DE)**
- **HOEFFKEN, Hans, Wolfgang**
  **D-6700 Ludwigshafen (DE)**
- **BOEHM, Hans-Joachim**
  **D-6703 Limburgerhof (DE)**
- **HORNBERGER, Wilfried**
  **D-6700 Ludwigshafen (DE)**

(56) Entgegenhaltungen:
**EP-A- 0 236 163**

- **PHARMAZIE Bd. 41, Nr. 4, 1986, BERLIN,DEUTSCHLAND Seiten 233 - 235; VOIGT ET AL: 'Synthese von N-alfa-(arylsulfonyl-l-prolyl)- und N-alfa-ben zyloxycarbonyl-l-prolyl)-d,l-4-amidinophenylala n inamiden als trombininhibitoren'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 668 869 B1

**Beschreibung**

Die Erfindung betrifft neue 2-[3-(4-Amidino-phenyl)]-propionsäurederivate, deren Herstellung sowie deren Verwendung zur Bekämpfung von Krankheiten.

Thrombin gehört zur Gruppe der Serinproteasen und spielt als terminales Enzym in der Blutgerinnungskaskade eine zentrale Rolle. Sowohl die intrinsische als auch die extrinsische Gerinnungskaskade führen über mehrere Verstärkungsstufen zur Entstehung von Thrombin aus Prothrombin. Die thrombinkatalysierte Spaltung von Fibrinogen leitet dann die Blutgerinnung und somit auch eine mögliche Thrombusbildung ein. Thrombin stimuliert außerdem die Aggregation der Thrombozyten, die ihrerseits durch die Bildung von Plättchenfaktor 3 und Gerinnungsfaktor XIII sowie eine ganze Reihe von hochaktiven Mediatoren die Thrombinbildung verstärken.

Thrombinbildung und -wirkung sind zentrale Ereignisse bei der Entstehung sowohl von weißen, arteriellen als auch von roten, venösen Thromben und daher potentiell wirksame Angriffspunkte für Pharmaka. Thrombin-Inhibitoren sind im Gegensatz zu Heparin in der Lage, unabhängig von Kofaktoren gleichzeitig die Wirkungen von Thrombin sowohl in der Gerinnungskaskade als auch auf Thrombozyten vollständig zu hemmen. Sie können in der Akutphase thromboembolische Ereignisse nach perkutaner transluminaler koronarer Angioplastie (PTCA) und Lyse verhindern und als Antikoagulantien in der extrakorporalen Zirkulation (Herz-Lungen-Maschine, Hämodialyse) dienen. Sie können auch allgemein zur Thromboseprophylaxe, beispielsweise nach chirurgischen Eingriffen dienen.

Als niedermolekulare Thrombin-Inhibitoren sind bisher zwei Klassen von Verbindungen beschrieben worden, nämlich

a) Substanzen vom Benzamidintyp (DD 155,954, FR 2.593.812, FR 2.593.813, EP 236.164, und C.A. 113, 6182j (1990) und

b) Substanzen vom Arginintyp (US 4,258,192, DE 2.801.478, EP 293.881, EP 185.390).

Es wurde nun gefunden, daß 2-[3-(4-Amidino-phenyl)]-propionsäurederivate der Formel

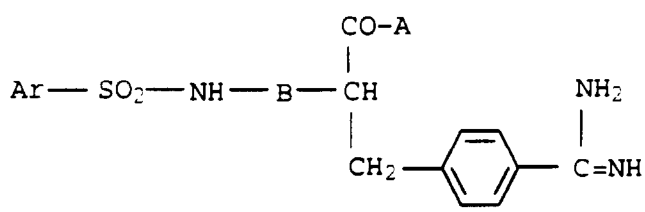

worin

A

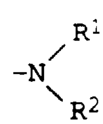

oder $-OR^3$ bedeutet, worin $R^1$ und $R^2$, die gleich oder verschieden sein können, für Wasserstoff, gesättigte oder ungesättigte Alkylreste mit bis zu 6 C-Atomen oder Aralkylreste oder Arylreste stehen, oder $R^1$ und $R^2$ zusammen mit dem sie verbindenden Stickstoffatom einen 5- bis 7-gliedrigen gesättigten Ring bedeuten, der gegebenenfalls durch Alkylreste mit bis zu 4 C-Atomen substituiert ist und der ein Sauerstoffatom enthalten kann, wobei die Stickstoff- und Sauerstoffatome in 1,2- bzw. 1,4-Stellung stehen,
und worin $R^3$ für Wasserstoff, einen gesättigten oder ungesättigten Alkylrest mit bis zu 6 C-Atomen, eine Cycloalkylgruppe oder eine Aralkylgruppe steht,

B

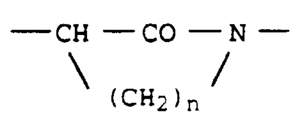

(mit n in der Bedeutung 1, 2, 3, 4 oder 5) oder die Gruppe

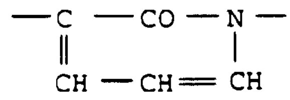

darstellt,

Ar eine Phenyl- oder α-, oder β-Naphthylgruppe bedeutet, die gegebenenfalls durch ein oder mehrere Halogenatome, Nitrogruppen, Aminogruppen, $C_{1-4}$-Mono- bzw. Bisalkylaminogruppen, Hydroxygruppen, $C_{1-4}$-Alkylreste, $C_{1-4}$-Alkoxygruppen, einen Methylendioxy- oder Ethylendioxyrest substituiert ist, oder Ar eine Pyridyl-, Chinolyl- oder Isochinolylgruppe bedeutet, die gegebenenfalls durch eine oder mehrere $C_{1-4}$-Alkylgruppen oder $C_{1-4}$-Alkoxygruppen substituiert sind,

sowie deren Salze mit physiologisch verträglichen Säuren eine bessere Wirkung besitzen.

Bevorzugt sind die Verbindungen, in denen B eine Pyrrolidinon-, eine Piperidinon- oder eine Piperidon-Gruppe bedeutet, A den Rest $-NR^1R^2$ darstellt, worin $R^1$ und $R^2$ zusammen mit dem Stickstoffatom einen 5- bis 7-gliedrigen Ring bedeuten, der durch $C_{1-4}$-Alkylreste substituiert sein kann, und Ar eine β-Naphthylgruppe darstellt, die durch ein oder mehrere $C_{1-4}$-Alkoxyreste substituiert ist.

Besonders bevorzugt sind solche Verbindungen, in denen A und B die vorstehend genannte Bedeutung haben und Ar eine β-Naphthylgruppe darstellt, die durch 1 oder 2 Methoxygruppen in der 4-, 5-, 6-, 7- und/oder 8-Stellung oder durch eine Methylendioxy- oder Ethylendioxygruppe in diesen Stellungen substituiert ist.

Die neuen Verbindungen besitzen ein bzw. zwei stereogene Zentren. Sie können sowohl als Racemate bezüglich der beiden stereogenen Zentren als auch als deren Antipoden vorliegen. Bevorzugt sind die Verbindungen, die am Phenylalaninbaustein R-konfiguriert sind.

Die neuen Verbindungen können gewünschtenfalls in Form ihrer Salze mit physiologisch verträglichen Säuren vorliegen. Als physiologisch verträgliche Säuren kommen insbesondere in Betracht: Salzsäure, Jodwasserstoffsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Die neuen Verbindungen werden hergestellt, indem man eine Verbindung der Formel II

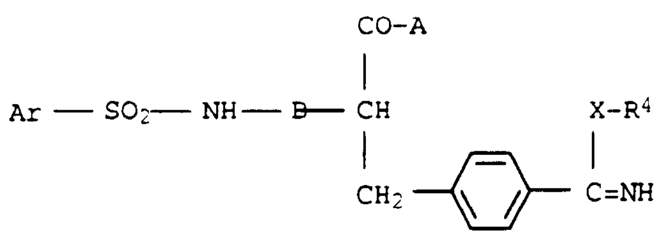

II,

worin $R^4$ $C_{1-3}$-Alkyl oder Benzyl und X ein Sauerstoff- oder Schwefelatom bedeuten, mit Ammoniak oder einem Ammoniumsalz umsetzt und die so erhaltenen Verbindungen gegebenenfalls in ihre Salze mit physiologisch verträglichen Säuren überführt.

Die Umsetzung von II erfolgt in der Regel bei 40 bis 80°C in einem polaren organischen Lösungsmittel, wie Tetrahydrofuran, Aceton, Dioxan, Methanol oder Dimethylformamid. Sie ist in der Regel nach 1 bis 10 Stunden beendet.

Die für die Herstellung der neuen Verbindungen benötigten Ausgangsstoffe sind noch nicht beschrieben worden. Sie lassen sich wie folgt gewinnen:

I Synthese des p-Cyanphenylalaninteils

a) Die Darstellung des p-Cyanphenylalanins ist in der DD-PS 155 954 beschrieben. Nach Alkylierung von Acetaminomalonester mit p-Cyanbenzylhalogeniden wird das entstehende Addukt durch Verseifung und Decarboxylierung zum p-Cyanphenylalanin umgesetzt, wobei durch partielle Hydrolyse der Nitrilfunktion schwer abtrennbare Nebenprodukte entstehen. Die nachfolgend beschriebenen Verfahren liefern den gewünschten p-Cyanphenylalaninbaustein in höherer Ausbeute und Reinheit.

b) Der p-Cyanphenylalaninbaustein wird dargestellt, indem man Isonitrilessigsäureester mit sekundären Aminen $HNR^1R^2$ zu den Verbindungen 2 umsetzt, wobei $R^1$ und $R^2$ die angegebene Bedeutung besitzen (siehe Synthesis 1977, 249)

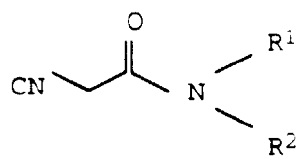

2

Diese Verbindungen ($R^1 \neq H$, $R^2 \neq H$) werden dann mit geeigneten Basen (z.B. Kalium-tert.-butylat, Butyllithium oder Lithiumdiisopropylamid) deprotoniert und mit p-Cyanbenzylverbindungen des Typs 3 umgesetzt. X stellt dabei eine nucleofuge Gruppe wie Halogen oder Alkyl- bzw. Arylsulfonat dar. Im Gegensatz zur Umsetzung von Isonitrilessigsäureestern mit p-Cyanbenzylhalogeniden erfolgt mit den Isonitrilessigsäureamiden 2 fast ausschließlich eine Monoalkylierung.

2 +

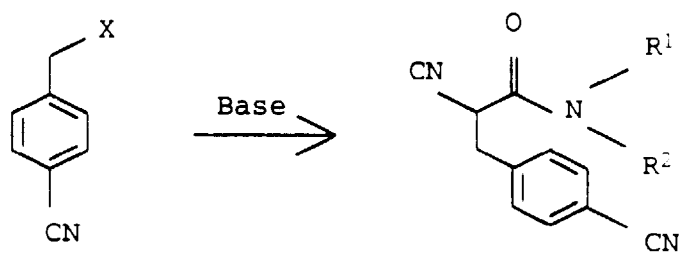


3 4

Anschließend wird die Isonitrilgruppe in 4 vorzugsweise unter wässrig sauren Bedingungen zum Amin 5 hydrolisiert

4

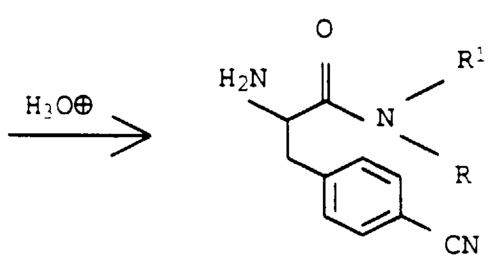


5

c) Die Umsetzung von N-(Diphenylmethylen)-glycinester ($R^4 = C_1$-$C_6$) 6 mit p-Cyanbenzylverbindungen 3 unter Verwendung geeigneter Basen ergibt das monoalkylierte Produkt 7, dessen Schutzgruppe unter sauren Bedingungen hydrolisiert wird (siehe J. Org. Chem. 47, 2663-2666 (1982). Bei Verwendung der Benzaldehydimine an Stelle der Benzophenonimingruppe erhält man mit p-Cyanbenzylbromid ein Gemisch aus mono- und bisalkyliertem Produkt

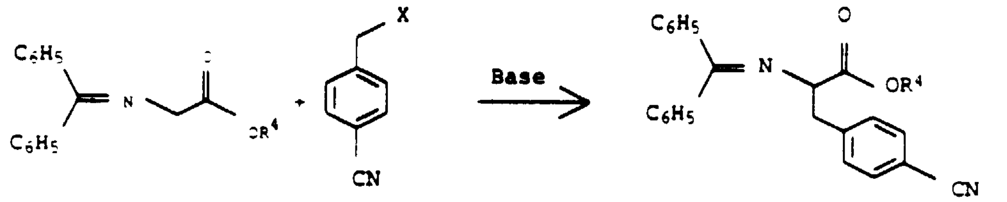


6 3 7

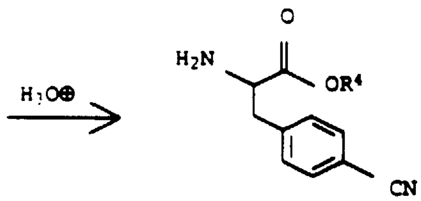


8

d) Die enantiomerenreinen Aminbausteine 5 und 8 können durch Racematspaltung mit optisch aktiven Säuren oder durch enantioselektive Synthesen gemäß e) oder f) gewonnen werden.

e) Die Umsetzung des Bislactimethers 9 ($R^5 = C_1$-$C_4$) mit p-Cyanbenzylverbindungen 3 nach bekannten Literaturvorschriften (Angew. Chem. 92, 205 (1980); Synthesis 1982, 864, 868; Tetrahedron 39 2085 (1983), Ann. Chem. 1983, 1133) ergibt die Verbindungen 10. Die saure Hydrolyse dieser Substanzen liefert die enantiomerenreinen p-Cyanphenylalaninester 11. Dabei entstehen aus den R-konfigurierten Lactimethern 9 die S-konfigurierten Alaninester 11 und umgekehrt, wobei bevorzugt die S-konfigurierten Verbindungen 9 eingesetzt werden.

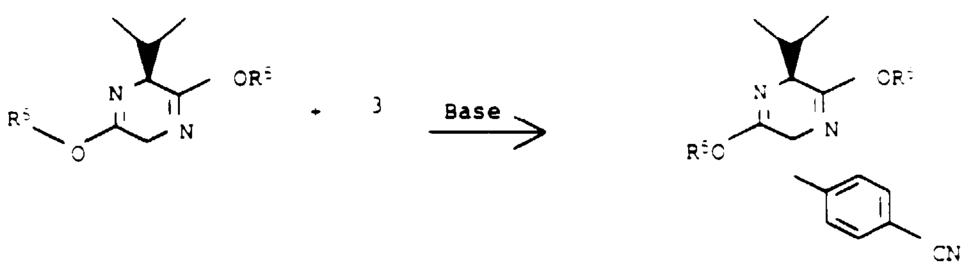


9 10

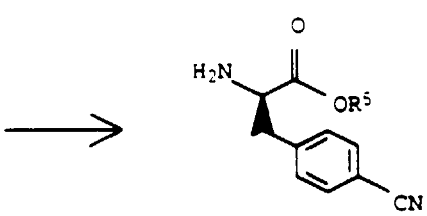

11

f) Bei der Umsetzung von Imidazolidinonen 12 mit p-Cyanbenzylverbindungen 3 in Analogie zu bekannten Literaturvorschriften (Tetrahedron 44 5277 (1988)) entsteht nach Hydrolyse der Zwischenprodukte 13 das p-Cyanphenylalanin 14

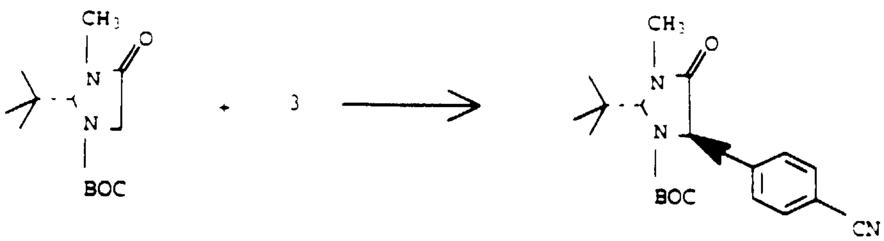

12

13

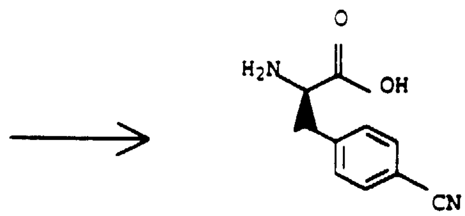

14

g) Bei Verwendung von chiralen Ketiminderivaten bei Methode c) z.B. Campherketiminderivat 15 entstehen die chiralen Alaninester 17 (siehe Synth. Comm. 19, 881-888 (1983))

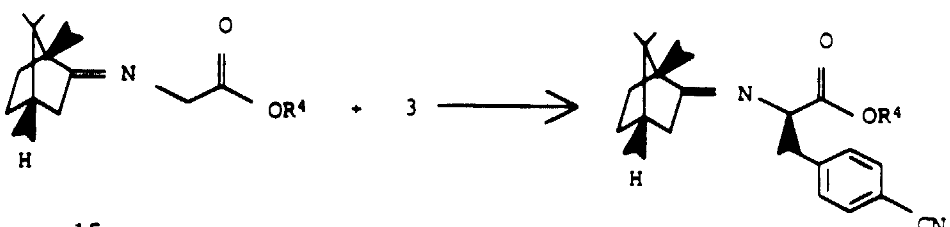

15

16

16

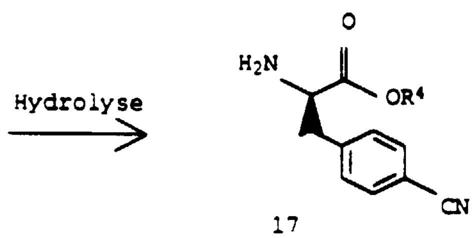


17

h) Wird die Aminofunktion im p-Cyanphenylalaninester, 8, 11, 17 mit einer Schutzgruppe vorübergehend geschützt, so lasen sich nach Hydrolyse der Esterfunktion und Aktivierung der Carbonsäure durch Umsetzung mit Alkoholen andere Ester darstellen, als auch durch Umsetzung mit Ammoniak bzw. primären oder sekundären Aminen die entsprechenden Amide 21 gewinnen, wobei $R^1$ und $R^2$ auch H sein können. Die Verbindungen 18 bis 21 können sowohl racemisch als auch enantiomerenrein sein.

Chemische Methoden zur Amidbindungsbildung sind ausführlich behandelt bei Müller, Methoden der Organischen Chemie Vol XV/2, pp 1 - 364, Thieme Verlag, Stuttgart, 1974; Bodanszky, Klausner, Ondetti, Peptide Synthesis, pp 85 - 128, John Wiley & Sons, New York, 1976 und anderen Standardwerken der Peptidchemie. Besonders bevorzugt sind

die Azidmethode, die symmetrische und gemischte Anhydridmethode, in situ erzeugte oder präformierte Aktivester und die Amidbindungsbildung mit Hilfe von Kupplungsreagenzien (Aktivatoren), insbesondere Dicyclohexylcarbodiimid (DCC), Diisopropylcarbodiimid (DIC), 1-Ethoxycarbonyl-2-ethoxy-1,2-dihydrochinolin (EEDQ), 1-Ethyl-3-(3-dimethylaminopropyl)-carbodiimidhydrochlorid (EDCI), n-Propanphosphonsäureanhydrid (PPA), N,N-Bis(2-oxo-3-oxazolidinyl) amidophosphorsaurechlorid (BOP-Cl), Diphenylphosphorylazid (DPPA), Castro's Reagenz (BOP), O-Benzotriazolyl-N,N,N',N'-tetramethyluronium-Salze (HBTU), 2,5-Diphenyl-2,3-dihydro-3-oxo-4-hydroxythiophendioxid (Steglichs Reagenz; HOTDO) und 1,1'-Carbonyl-diimidazol (CDI). Die Kupplungsreagenzien können allein oder in Kombination mit Additiven wie N,N'-Dimethyl-4-aminopyridin (DMAP), N-Hydroxybenzotriazol (HOBt), N-Hydroxybenzotriazin (HOOBt), N-Hydroxysuccinimid (HOSu) oder 2-Hydroxypyridin eingesetzt werden.

Bei den Peptidsynthesen werden drei literaturbekannte Schutzgruppentechniken bevorzugt: Die Benzyloxycarbonyl(Z)-, die t-Butyloxycarbonyl(Boc) - und die 9-Fluorenylmethyloxycarbonyl(Fmoc)-Schutzgruppentechnik.

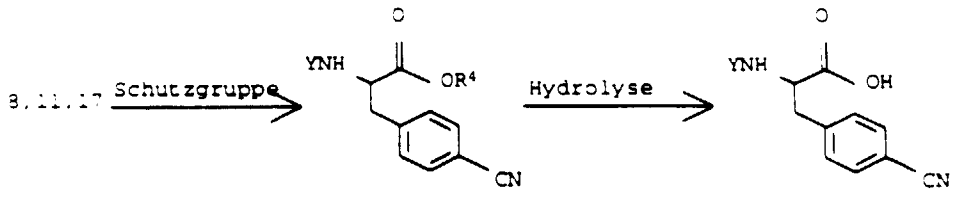


18 19

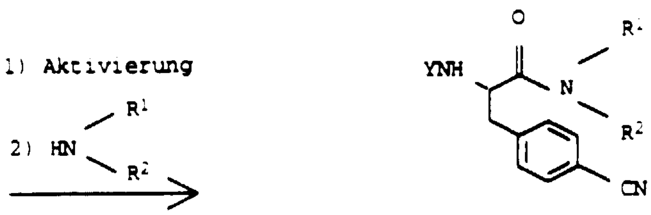


20

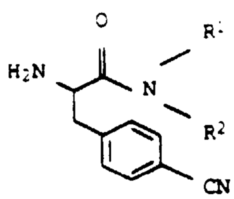

21

II Synthese der Pyrrolidinone

a) Methioninderivate als Edukte

Die Amine 5, 8, 11, 17 und 21, nachfolgend vereinfacht nur mit 22 ($R^3 \neq$ H, A wie in Formel I) gekennzeichnet, werden unter den in der Peptidchemie üblichen Bedingungen (siehe oben) mit N-geschütztem Methionin 23 zu 24 umgesetzt. Y steht für eine Cbz, BOC oder Fmoc-Schutzgruppe.

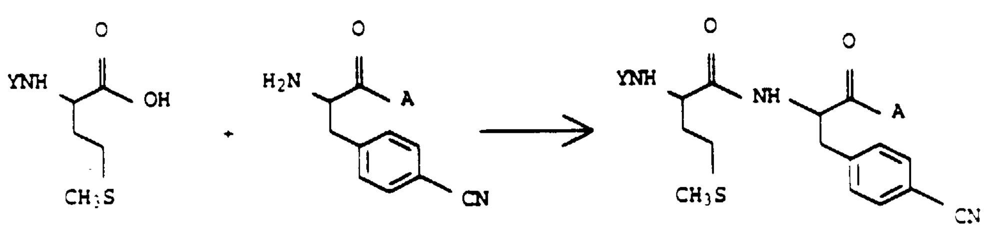

23 22 24

Das durch Alkylierung von 24 mit einem Alkyl- oder Aralkylhalogenid R'X, vorzugsweise Methyljodid bzw. Benzylbromid, erhaltene Sulfoniumsalz 25 läßt sich mit geeigneten Basen zum Pyrrolidinonring 26 cyclisieren. Als Basen eignen sich Natriumhydrid, Kaliumtert.-butylat, Butyllithium, Lithium-bis-(trimethylsilyl)amid, Lithiumdiisopropylamid, wobei letzteres bevorzugt wird. Die Abspaltung der Schutzgruppe ergibt das Amin 27

24 + R'X

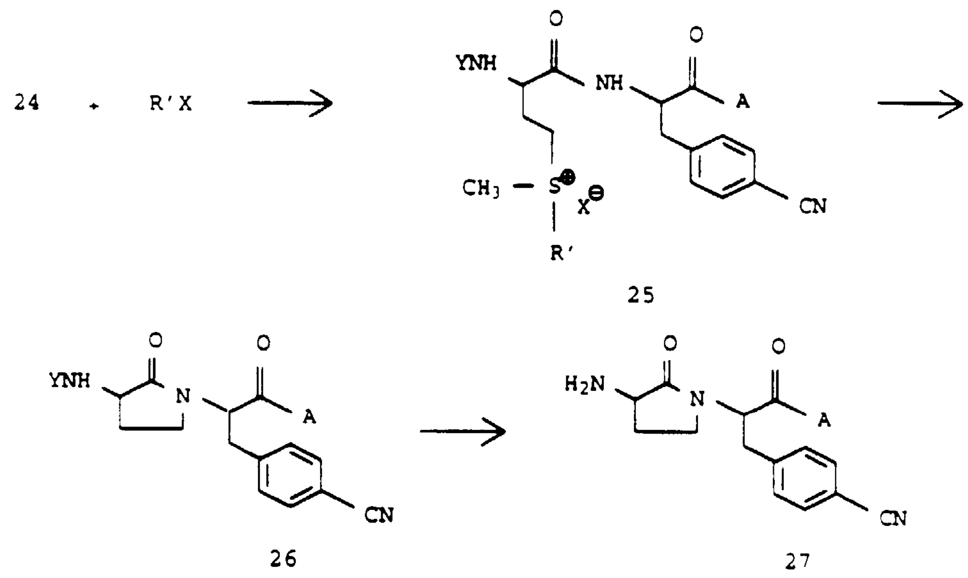

25

26

27

b) Asparaginsäurederivate als Edukte

Die Umsetzung des N-geschützten Asparaginsäurederivats 28

($R^5$ = $C_1$-$C_4$-Alkyl, Benzyl) mit dem Amin 5 bzw. 21 unter den in der Peptidchemie üblichen Bedingungen (siehe oben) ergibt das Zwischenprodukt 29, dessen Esterfunktion selektiv zum Alkohol reduziert und in eine Abgangsgruppe X (z.B. Halogen-, Mesylat-, Triflat, Tosylatgruppe) umgewandelt wird. Bei Behandlung mit geeigneten Basen (analog zu Methionin siehe oben) erhält man die Pyrrolidinone 32, die anschließend entschützt werden.

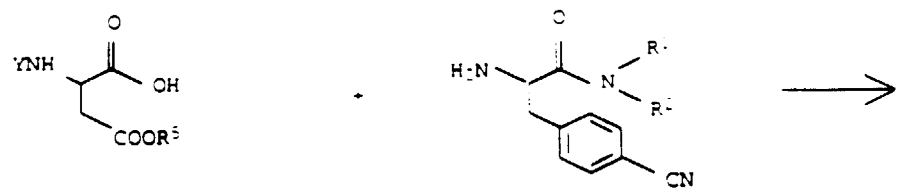

28 5.21

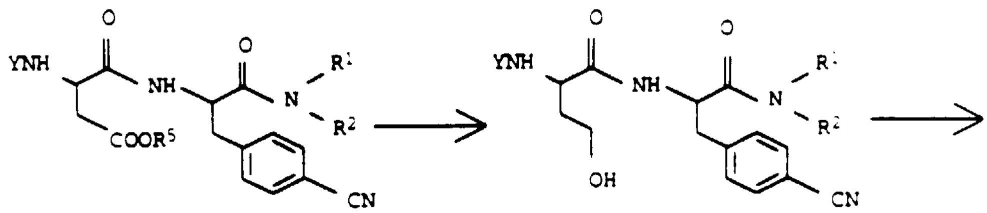

29 30

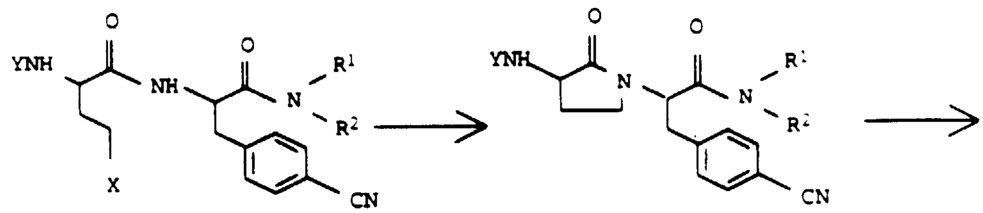

31 32

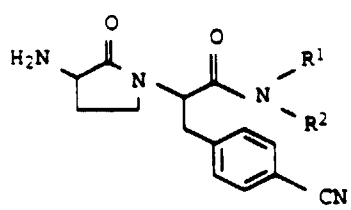

33

c) Oxazolidinonaldehydderivate als Edukte

Die Umsetzung von geschützten Derivaten 34 deren Synthese für Y' = Cbz beschrieben ist (TH 42 6551 (1986), mit den Aminen 22 ($R^3 \neq H$) unter reduktiven Bedingungen ergibt die Verbindungen 35, welche mit Basen in die geschützten Pyrrolidinone 36 überführt werden. Bei Verwendung der Cbz-Schutzgruppe wird diese anschließend hydrogenolytisch abgespalten. Y' kann aber auch bereits eine $ArSO_2$-Gruppe darstellen, die somit im Molekül verbleibt. Die Oxazolidinonaldehydderivate 34 werden jeweils enantiomerenrein eingesetzt.

Y' = $ArSO_2$, Cbz

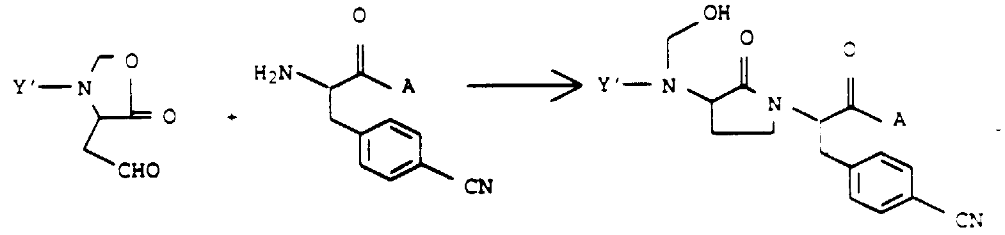

34 22 35

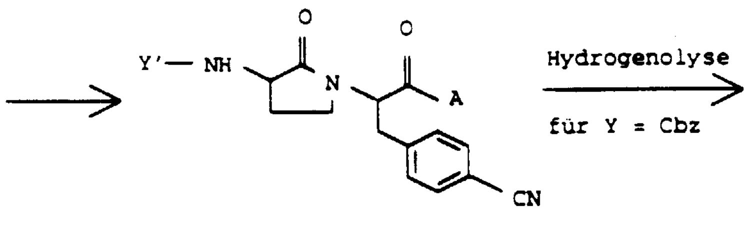


27

36

III Synthese der Piperidinone

a) Glutaminsäurederivate als Edukte

In Analogie zu der Darstellung der Pyrrolidonone 33 ausgehend vom N-geschützten Asparaginsäurederivat 28 lassen sich die entsprechenden Piperidinone 39 darstellen, wenn man als Edukt das homologe Glutaminsäurederivat 37 einsetzt.

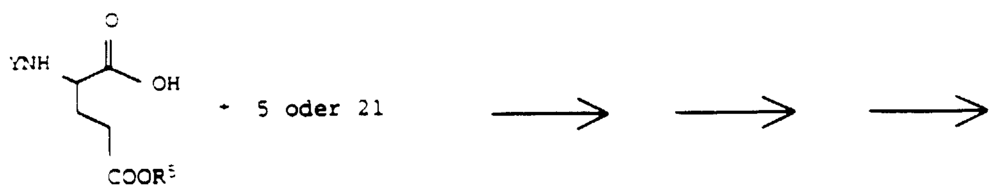


37

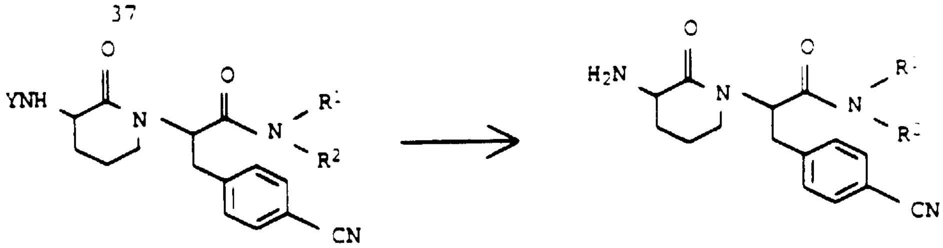

38 39

b) α-Amino-valeriansäurederivate mit einer δ-ständigen Abgangsgruppe als Edukte

Alkyliert man N-substituierte p-Cyanphenylalaninderivate 20 ($R^1 \neq H$, $R^2 \neq H$, Y = Cbz, $CF_3CO$) mit α-Amino-valeriansäurederivaten 40 (X = Halogen, Mesylat-, Triflat-, Tosylatgruppe; für X = Br, Y' = BOC, $R^4 = C_4H_9$: J. Org. Chem. 49, 3527 (1984)) mit Hilfe geeigneter Basen, so erhält man die Zwischenprodukte 41, welche sich nach Abspaltung der Schutzgruppe Y zu den Piperidinonen 38 cyclisieren lassen

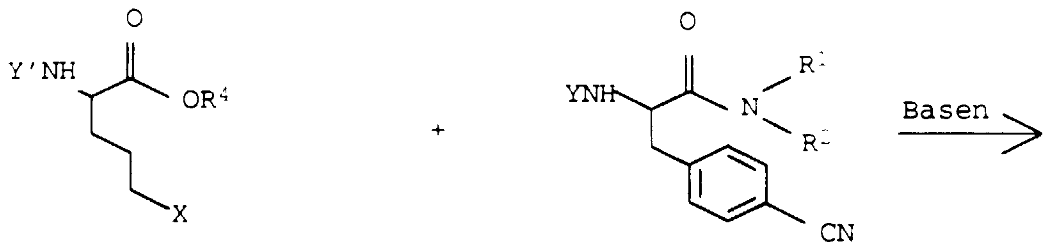


40 20

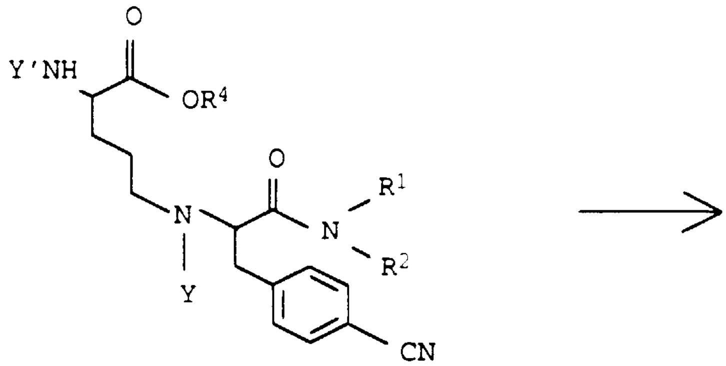

38

41

c) Oxazolidinonaldehydderivate als Edukte.

Setzt man in Analogie zu IIc die homologen Oxazolidinonaldehydderivate 42 (für Y = Cbz: Tetrahedron Lett. 25, 927 (1984)) mit den Aminen 22 $R^3 \neq H$ um, so erhält man die entsprechenden Piperidinone 44 bzw. 45 Ist Y' eine $ArSO_2$-Gruppe, so verbleibt diese Gruppe im Molekül.

Y' = $ArSO_2$, Cbz

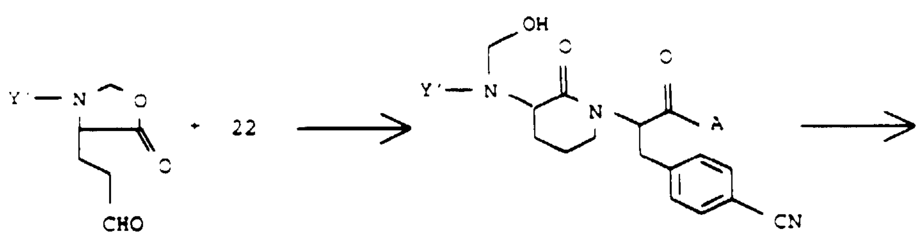

22

42 43

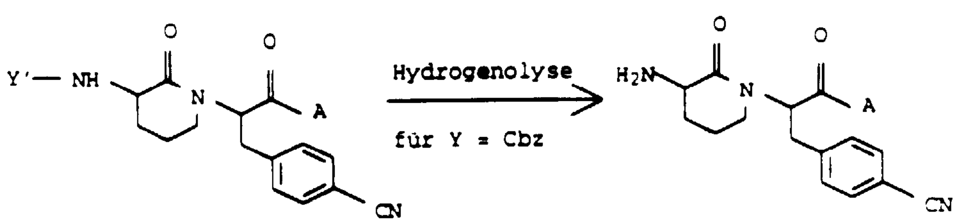


44 45

d) Ornithinderivate als Edukte

Die Kondensation des 3(p-Cyanphenyl)-brenztraubensäureamids 46 mit dem N geschützten Ornithinderivat 47 ($R^6$ = $C_1$-$C_4$) und anschließender Reduktion der Imingruppe ergibt das Zwischenprodukt 49, welches sich zum Piperidinon 38 cyclisieren läßt

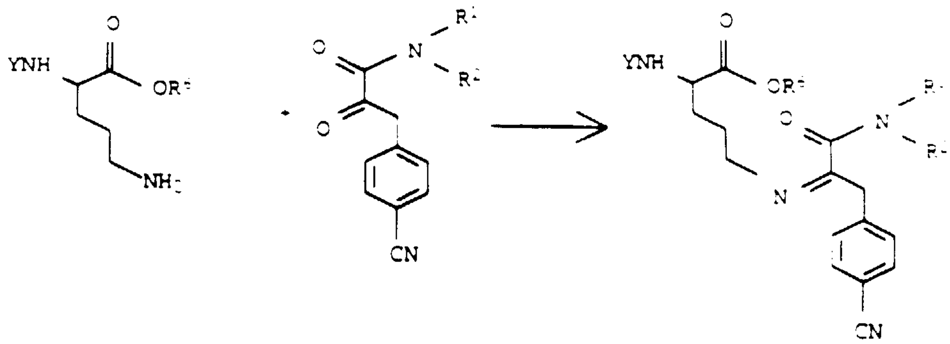

47 46 48

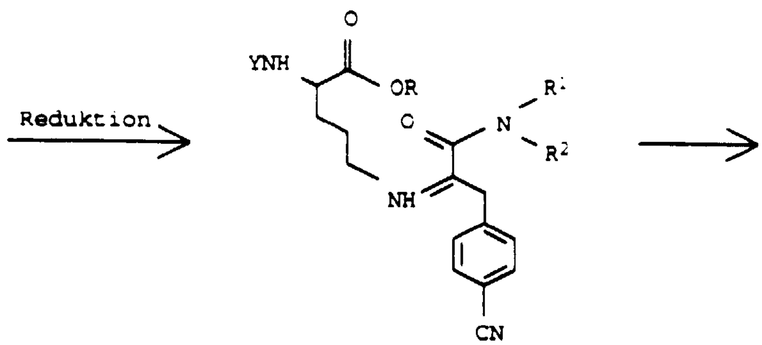


38

49

e) Isonitrilessigsäureester als Edukt

In Analogie zur Umsetzung von Isonitrilessigsäureester mit Piperidin (Ib) ergibt sich bei der Umsetzung mit dem Amin 5 bzw. 21 ($R^1 \neq H$, $R^2 \neq H$) das Zwischenprodukt 51, welches mit 1.3-Dihalogenalkanen ($X^1$, $X^2$ = Hal) unter Einwirkung von Basen zum Piperidinon 52 cyclisiert werden kann. Dessen Isonitrilgruppe kann durch wässrig saure Hydrolyse ins Amin 39 überführt werden.

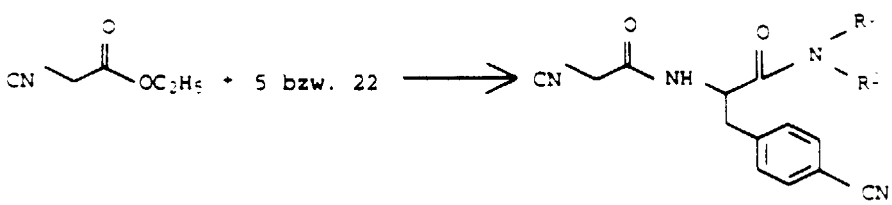


51

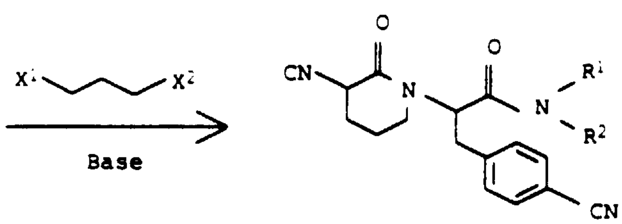


39

52

IV Umsetzung der Pyrrolidone und Piperidinone mit aromatischen Sulfonsäurechloriden

Die Zwischenstufen 27, 33, 39 und 45 lassen sich mit den aromatischen Sulfonsaurechloriden $ArSO_2Cl$ unter Basenkatalyse zu den Sulfonamiden 53 und 54 umsetzen

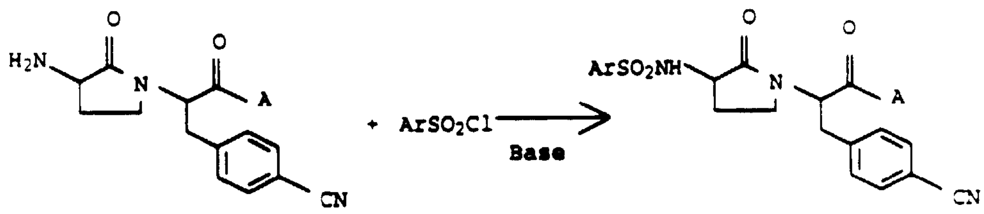


27, 33 53

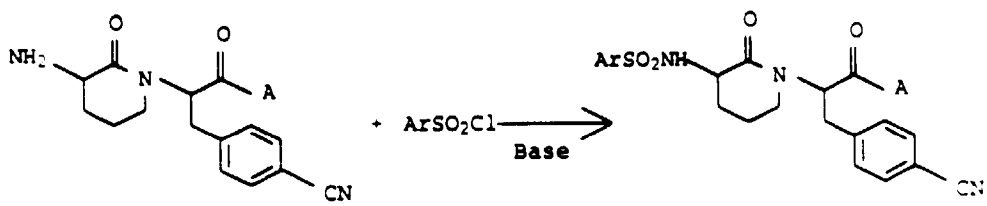


39,45 54

V Synthese der Pyridone

Ausgehend von 3-Nitro-2-hydroxy-pyridin 55 erhält man durch N-Alkylierung mit α-Halogenessigsäureestern ($R^4$=$C_1$-$C_6$, X=Cl, Br) 56 die Pyridone 57. Verwendet man den entsprechenden tert.-Butylester ($R^4$=$C(CH_3)_3$), so läßt sich dieser im Pyridon 57 leicht mit Trifluoressigsäure spalten. Die Carbonsäure 58 wird nach Aktivierung mit den Aminen $HNR^1R^2$ ($R^1$, $R^2 \neq H$) zu den Zwischenverbindungen 59 umgesetzt. Diese können auch direkt durch Umsetzung von 55 mit α-Halogenessigsäureamiden ($R^1$, $R^2 \neq H$, X=Cl, Br) 60 dargestellt werden. Die katalytische Reduktion der Nitrofunktion in 59 ergibt die Aminopyridone 61. Diese werden zunächst mit den aromatischen Sulfonsäurechloriden $ArSO_2Cl$ und anschließend zum Schutz der $SO_2NH$-Funktion für die nachfolgende Alkylierung mit Di-tert.-butyldicarbonat zu den Zwischenprodukten 63 umgesetzt. 63 wird nach Deprotonierung (man verwendet vorzugsweise Lithiumdiisopropylamid) mit p-Cyanbenzylhalogenid 3 zum Pyridon 64 alkyliert. Die BOC-Schutzgruppe kann anschließend oder aber nach Umwandlung der Nitrilin die Amidinfunktion (siehe Abschnit VII) mit Trifluoressigsäure abgespalten werden.

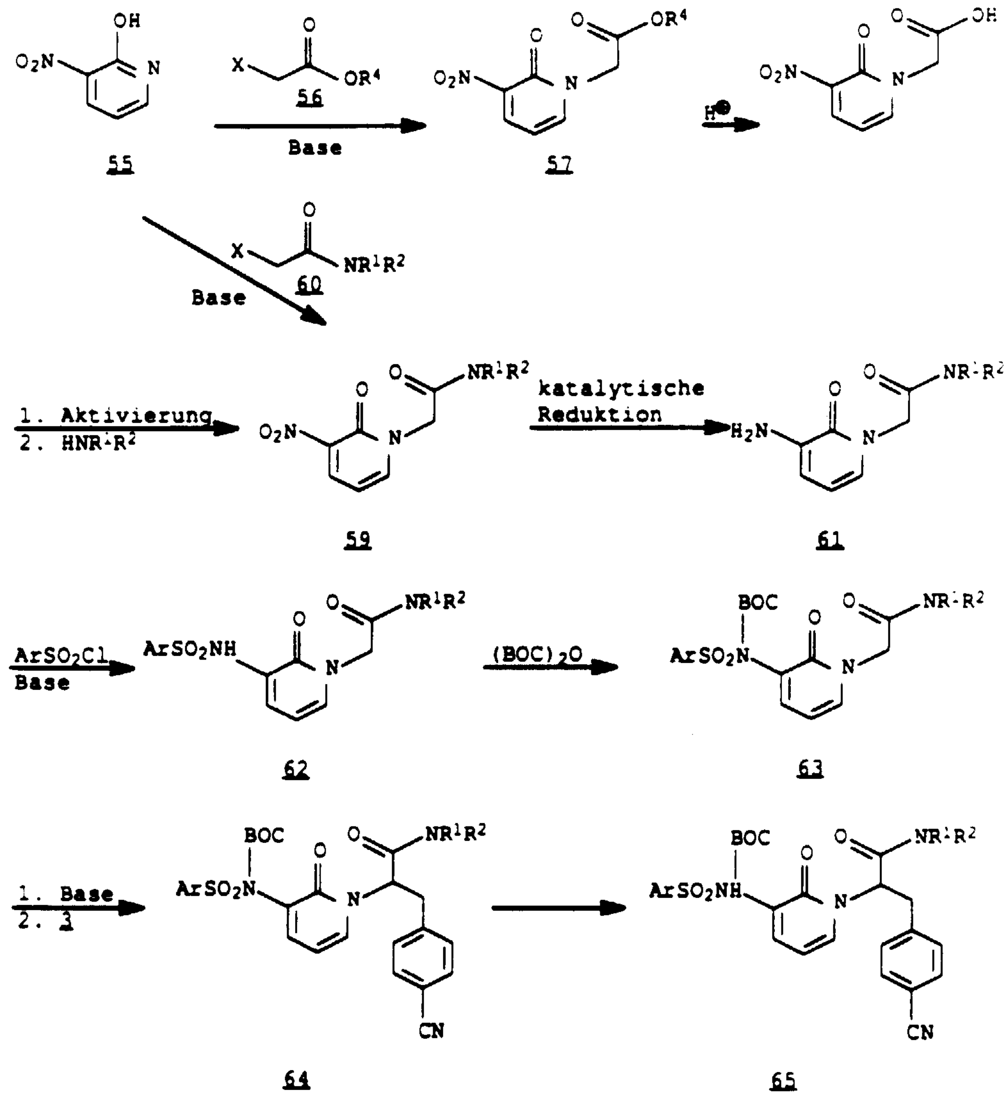


Weiterhin ist es möglich, in analoger Weise den Ester 57 in den Ester 66 bzw. 67 zu überführen:

57

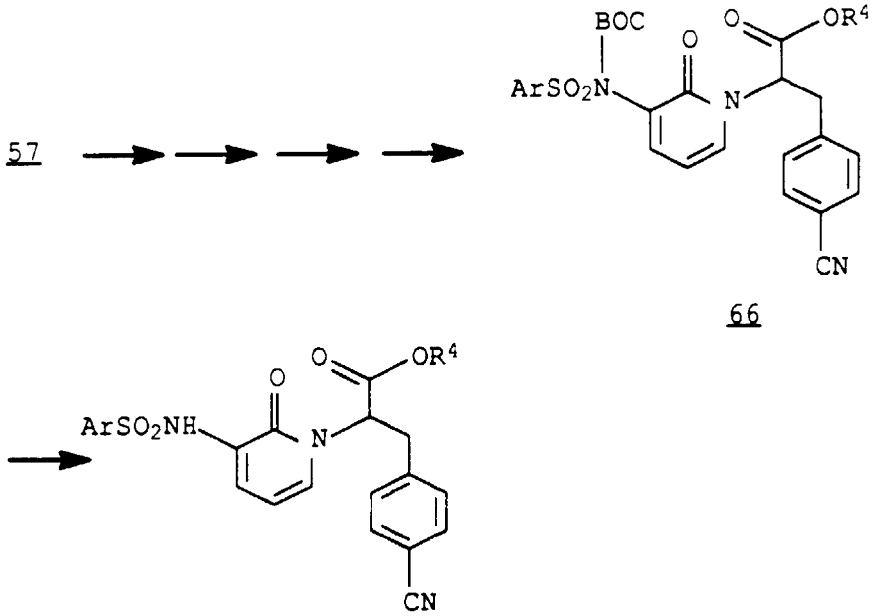

66

67

VI Umwandlung der Esterfunktionen in den Zwischenprodukten 26, 36, 44, 53, 54, 66 und 67.

In Analogie zu Ih lassen lassen nach Hydrolyse der Esterfunktionen in 26, 36, 44, 53 und 54 (A = $OR^3$ $R^3 \neq H \rightarrow R^3 = H$) bzw. 66 und 67 ($R^4 \neq H, \rightarrow R^4 = H$), Aktivierung der Carbonsäuren und anschließende Umsetzung mit Alkoholen andere Ester (A = $OR^3$) und durch Umsetzung mit Aminen ($HNR^1R^2$) die entsprechenden Amide (A = $NR^1R^2$) darstellen.

VII Umwandlung der Nitrilfunktion in die Amidinfunktion.

Die Umwandlung der Nitrilfunktion in 53, 54, 65 und 67 erfolgt in typischer Weise einerseits über die Pinner-Variante durch säurekatalysierte Addition von Alkoholen ($R^7 = C_1$-$C_4$) unter Bildung der Iminoether 68, welche mit Ammoniak bzw. Ammoniumsalzen die entsprechenden Amidine I bilden. Andererseits läßt sich die Amidingruppe in I auch erzeugen, indem man Schwefelwasserstoff an die Nitrilfunktion in 53, 54, 64, 65, 66 bzw. 67 addiert, die entstehende Thioamidgruppe 69 bzw. 70 mit Alkylhalogeniden (X = Hal, $R^8$ - $C_1$-$C_3$ Alkyl, Benzyl) am Schwefel alkyliert und die Iminothioether 71 bzw. 72 mit Ammoniak bzw. Ammoniumsalzen umsetzt. Die BOC geschützten Amidinderivate lassen sich durch Abspaltung der Schutzgruppe (vorzugsweise mit Trifluoressigsäure in Methylenchlord) in die Endprodukte I überführen.

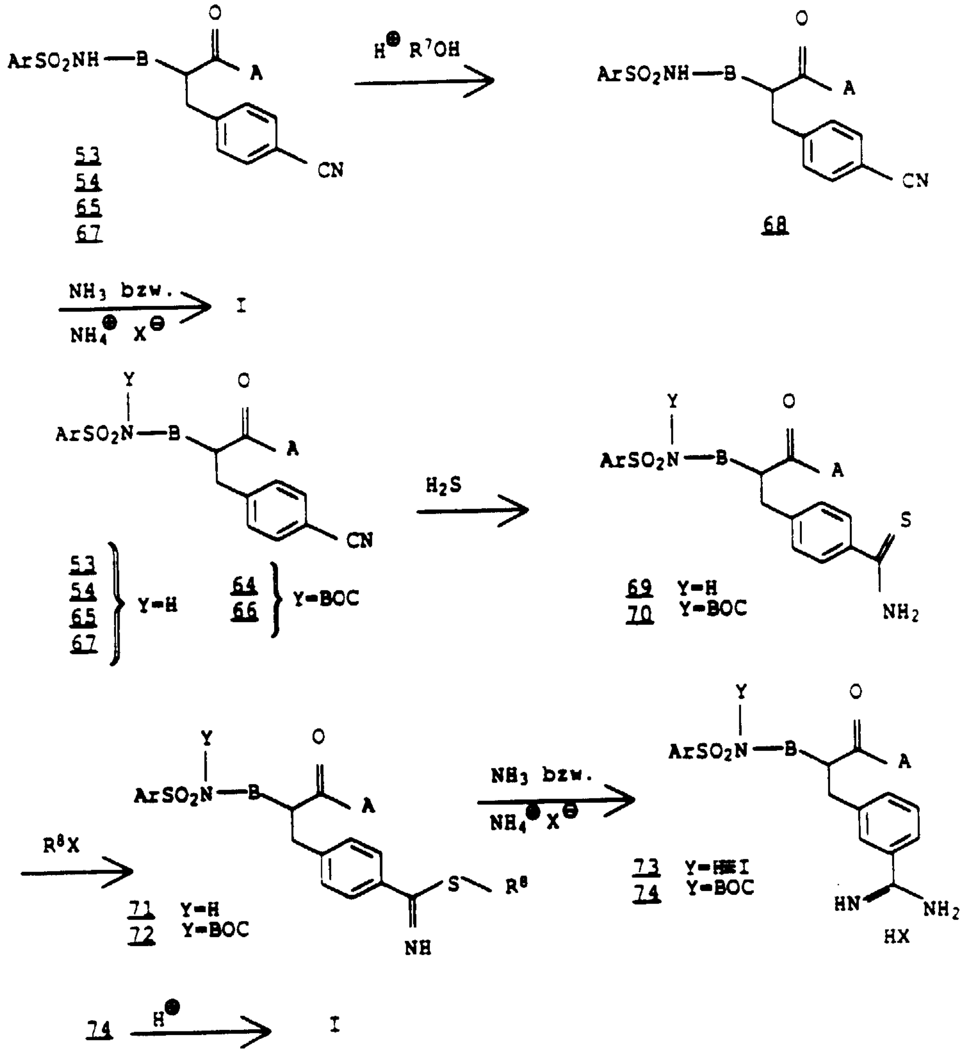


Die erfindungsgemäßen Verbindungen besitzen eine gute Thrombin-inhibitorische Wirkung, ohne andere Serinproteasen (z.B. Trypsin und Plasmin) wesentlich zu beeinflussen. Sie lassen sich daher zur Prophylaxe und Therapie thromboembolischer Erkrankungen, wie Herzinfarkt, Thrombosen und Embolien, verwenden.

Die pharmakologische Charakterisierung der neuen Verbindungen als Thrombin-Inhibitoren erfolgte in folgenden Testsystemen:

1. Plasma-Thrombinzeit (PTZ) in vitro

Zur Gewinnung von Citratplasma wird humanes Blut mit Natriumcitrat (0,11 mol/l) gemischt (9 Teile Blut + 1 Teil Natriumcitrat) und anschließend 10 min bei 1600 xg bei Raumtemperatur zentrifugiert. Zu 50 µl der Substanzlösung bzw. Lösungsmittel werden 50 µl Citratplasma gegeben und 2 min bei 37°C inkubiert. Danach werden 100 µl auf 3°C temperiertes Thrombin-Reagenz (Boehringer Mannheim) zupipettiert und die Zeit bis zum Eintritt der Gerinnung in einem photometrischen Koagulometer gemessen.

Als Maß der relativen Wirksamkeit wird als $EC_{100}$ die Konzentration einer Prüfsubstanz in mol/l berechnet, die die Plasma-Thrombinzeit um 100 % verlängert.

Die Plasma-Thrombinzeit erfaßt die Thrombin-induzierte Fibrinbildung aus Fibrinogen und die Fibrinaggregation, d.h. den letzten Schritt der Gerinnung.

2. Amidolytische Bestimmung der Aktivität von Thrombin und anderen Serinproteasen (Trypsin, Chymotrypsin, Plasmin, Faktor Xa, aktivtes Protein C)

Testprinzip:

chromogenes Substrat $\xrightarrow{\text{Serin-protease}}$ Peptid + p-Nitroanilin (gelb)

In Mikroplatten werden jeweils 250 µl Thrombin (0,124 IU/ml, EK 0,1 IU/ml) in Tris-Puffer (Tris 50 mmol/1, NaCl 154 mmol/l, pH 8,0) vorgelegt. Diese Lösung wird mit 10 µl Lösungsmittel (Kontrolle) bzw. Prüfsubstanz versetzt, 1 min gemischt und 4 min bei 25°C inkubiert. Danach wird die Reaktion durch Zugabe von 50 µl Substratlösung (S-2238, 0,62 mmol/l, EK 0,1 mmol/l) gestartet und nach kurzem Mischen bei 25°C inkubiert. Nach 5 min wird die Reaktion durch Zugabe von 50 µl 35 %iger Essigsäure gestoppt und die Extinktion bei 405 nm (gegen 630 nm) gemessen. Die nach Reaktionsende gemessene Extinktion ist proportional der Enzymaktivität.

Als Maß der relativen Wirksamkeit wird als $IC_{50}$ die Konzentration einer Prüfsubstanz in mol/l berechnet, die die Enzymaktivität um 50 % vermindert.

Analog zu Thrombin wird auch die Wirkung auf die amidolytische Aktivität von anderen Serinproteasen

| | |
|---|---|
| Trypsin (0,1 mg/l) | mit S-2222 (0,1 mmol/l) |
| Chymotrypsin (0,2 mg/l) | mit S-2586 (0,1 mmol/l) |
| Plasmin (0,04 CU/ml) | mit S-2251 (0,1 mmol/l) |
| Faktor Xa (0,2 nkat/ml) | mit S-2765 (0,1 mmol/1) |
| aktives Protein C | mit S-2366 (0,2 mmol/1) |

(jeweils Endkonzentration im Test) untersucht. Die Ergebnisse dieser Tests ermöglichen eine Aussage über die Selektivität der Wirkung von Prüfsubstanzen.

3. Thrombininduzierte Thrombozytenaggregation in vitro

Frisches humanes Citratblut (9 Teile Blut + 1 Teil Natriumcitrat 0,11 mol/l) wird zur Gewinnung von plättchenreichem Plasma (PRP) und plättchenarmem Plasma (PPP) 16 min bei 250 xg bzw. 20 min bei 3670 xg zentrifugiert.

Zur Bestimmung der Thrombozytenaggregation werden 445 µl PRP mit 5 µl Lösungsmittel (Kontrolle) bzw. Prüfsubstanz versetzt und 5 min bei Raumtemperatur inkubiert. Danach wird der Ansatz 3 min bei 37°C in einem Aggregometer (ELVI 840) inkubiert und 4 min bei 1000 RPM gerührt. Die Aggregation wird durch Zugabe von 50 µl Thrombinlösung (Endkonzentration im Ansatz: 0,15 IU/ml) gestartet. Die Thrombozytenaggregation wird über die Änderung der gemessenen Transmission pro Zeiteinheit in der Probe bestimmt (Slope-Methode).

Als Maß der relativen Wirksamkeit wird als $IC_{50}$ die Konzentration einer Prüfsubstanz in mol/l berechnet, die die Thrombozytenaggregation um 50 % hemmt.

4. Antithrombotische Wirkung im arteriovenösen Shunt an der Ratte

In diesem Experiment wird in einem arteriovenösen Shunt durch eine Glaskapillare eine künstliche Thrombose ausgelöst.

Die narkotisierte (Urethan 25 %, 2 x 8 mg/kg i.p.) Ratte wird in Rückenlage auf einer temperierten (37°C) Wärmebank fixiert. In die freipräparierte rechte A. carotis und V. jugularis werden kurze Polyethylene-Katheter (Portex, PE 50) implantiert, mit physiologischer NaCl-Lösung gefüllt und durch Klemmen verschlossen. Die freien Enden der Katheter werden durch eine 20,0 mm lange Glaskapillare (Innendurchmesser 1,0 mm) verbunden, die als thrombogene Oberfläche wirkt.

Die Applikation der Prüfsubstanz kann i.v., s.c., p.o. oder als Infusion erfolgen. Nach der gewünschten Inkubationszeit (5, 60 oder 360 min) mit der Prüfsubstanz oder Lösungsmittel (Kontrolle) wird der Shunt durch Entfernen der Klemmen geöffnet. Der Blutstrom durch den Shunt führt zu einem schnellen Anstieg der Shunttemperatur, die an der Mitte der Glaskapillare gemessen wird. Die Erhöhung von Raumtemperatur auf Körpertemperatur ist ein Indikator für die Durchgängigkeit des Shunts. Die Temperatur wird bis zum Verschluß des Shunts, längstens jedoch 30 min, kontinuierlich aufgezeichnet.

Bei Öffnung des Shunts und am Ende des Experiments werden zusätzlich Blutproben zur Bestimmung der anti-FIIa-Aktivität im Plasma entnommen.

Die Versuchsauswertung erfolgt quantitativ. Mit den Werten von log Dosis und der Zeit (als Differenz der Verschlußzeiten von behandelter Gruppe und Kontrollgruppe) wird eine lineare Regression berechnet. Für den Vergleich der Wirksamkeit unterschiedlicher Testsubstanzen wird aus der Gleichung der Regressionsgeraden der Wert für die ED 15 min (die Dosis, die bezogen auf die Kontrollguppe zu einer Verlängerung der Verschlußzeit um 15 min führt) berechnet.

In diesen Tests zeigen die neuen Verbindungen sehr gute Eigenschaften.

Die neuen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 10 und 1000 mg/kg Körpergewicht bei oraler Gabe und zwischen etwa 1 und 100 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gewichtsprozent.

## Experimenteller Teil

Alle Reaktionen wurden unter Inertgas, vorzugsweise Stickstoff, durchgeführt.

Die für die Reaktionen verwendeten Lösungsmittel wurden über Molekularsiebe getrocknet.

### Beispiel 1

a) Darstellung von α-Isocyanessigsäurepiperidid

Zu 25,8 g (228 mmol) Isocyanessigsäureethylester in 200 ml trockenem Methanol wurden 48,7 g (570 mmol) trokkenes Piperidin gegeben und über Nacht bei Raumtemperatur gerührt. Die Reaktionsmischung wurde anschließend zur Trockene eingeengt und mit Ether/Isopropanol kristallisiert. Man erhielt 33 g (≙ 95 % d.Th.) reines α-Isocyanessigsäurepiperidid als beige Festsubstanz.

b) Darstellung von 3 (p-Cyanphenyl)-2-isocyanpropionsäurepiperidid

22,0 g (144,5 mmol) α-Isocyanessigsäurepiperidid in 160 ml THF wurden bei -70°C zu 159 mmol Lithiumdiisopropylamid in 430 ml THF getropft. Anschließend tropfte man 28,3 g (144,5 mmol) p-Cyanbenzylbromid - gelöst in 300 ml THF - bei -70°C zur Reaktionsmischung, rührte 3 h bei dieser Temperatur (DC-Kontrolle, Fließmittel $CH_2Cl_2/CH_3OH$ 9/1) und versetzte nach Reaktionsende tropfenweise mit 70 ml Wasser. Die Lösung wurde im Vakuum eingeengt, der Rückstand mit 1N Salzsäure angesäuert, mit Methylenchlorid extrahiert, die organische Phase über Magnesiumsulfat getrocknet und einrotiert. Das Festprodukt wurde mit Ether versetzt, über Nacht gerührt und am folgenden Tag abgesaugt. Man erhielt 30,9 g (plus 3,3 g durch Aufarbeitung der Mutterlauge) 3-(p-Cyanphenyl)-2-isocyan-propionsäurepiperidid als wäßrige Festsubstanz (86 % d.Th.).

c) Darstellung von p-Cyanphenylalaninylpiperidid-hydrochlorid

17,4 g (59,2 mmol) 3(p-Cyanphenyl)-2-isocyanpropionsäurepiperidid wurden zusammen mit 160 ml Dioxan und 24 ml konzentrierter Salzsäure 45 bis 60 min bei 60°C gerührt (DC-Kontrolle, Fließmittel $CH_2Cl_2/CH_3OH$ 9/1), anschließend wurde das Reaktionsgemisch im Vakuum eingeengt, der Rückstand in Wasser aufgenommen, mit wenig Essigester extrahiert, die wäßrige Phase mit Ammoniak versetzt, mit Methylenchlorid extrahiert, über Magnesiumsulfat getrocknet und im Vakuum eingeengt. Der Rückstand wurde in Aceton aufgenommen und das Hydrochlorid mit Salzsäure in Ether gefällt. Der Niederschlag wurde abgesaugt und mit Ether gewaschen. Man erhielt 16,3 g (≙ 83 %) p-Cyanphenylalaninylpiperididhydrochlorid als weiße Festsubstanz.

d) Darstellung von BOC-L-(S-methyl)-methionyl-D,L-4-cyanphenylalaninylpiperidid-jodid

11,2 g (22,92 mmol) BOC-L-methionyl-D,L-4-cyanphenylalaninylpiperidid wurden zusammen mit 35 ml Methylenchlorid und 45 ml Methyljodid im verschlossenen Kolben 4 Tage bei Raumtemperatur gerührt. Methylenchlorid und überschüssiges Methyljodid wurden zunächst im Wasserstrahl- und anschließend im Hochvakuum bei Raumtemperatur vollständig entfernt. Der Rückstand wurde ohne weitere Aufreinigung in die nachfolgend unter e) beschriebene Cyclisierung eingesetzt.

e) Darstellung von 3(tert.-Butyloxycarbonylamino)-1-(2-(3-(4-cyanphenyl)propionsäurepiperidid))-pyrrolidin-2-on

Zu der bei -70°C dargestellten Lösung von 65,9 mmol Lithiumdiisopropylamid in 300 ml THF tropfte man das Produkt des oben beschriebenen Ansatzes d) gelöst in 100 ml THF. Nach 30 min Reaktionszeit bei -70°C ließ

man langsam auf Raumtemperatur erwärmen und rührte über Nacht bei dieser Temperatur. Danach wurde die Reaktionsmischung im Vakuum eingeengt, der Rückstand in Ether aufgenommen, mit 1N Salzsäurelösung gewaschen, der in Ether unlösliche Anteil abfiltriert, die Etherphase mit Wasser gewaschen, über Magnesiumsulfat getrocknet und im Vakuum eingeengt. Man erhielt 8,7 g (≙ 86 %) leicht verunreinigtes 3(tert.-Butyloxycarbonylamino)-1-(2-(3-(4-cyanphenyl)propionsäurepiperidid))-pyrrolidin-2-on, welches ohne weitere Reinigung weiter eingesetzt wurde.

f) Darstellung von 3-Amino-1-(2-(3-(4-cyanphenyl)-propionsäurepiperidid))pyrrolidin-2-on (als Trifluoracetat)

Das gemäß e) erhaltene Rohprodukt wurde zusammen mit 43 ml Methylenchlorid und 23 ml Trifluoressigsäure 90 min bei Raumtemperatur gerührt. Anschließend wurde das Reaktionsgemisch zunächst im Wasserstrahlvakuum, dann im Hochvakuum bei Raumtemperatur eingeengt. Das entstandene mäßig verunreinigte Produkt 3-Amino-1-(2-(3-(4-cyanphenyl)-propionsäurepiperidid))pyrrolidin-2-on (als Trifluoracetat) wurde ohne weitere Reinigung in die nächste Stufe eingesetzt.

g) Darstellung von 3-(6,7-Dimethoxy-naphthalin-2-sulfonylamino) -1-(2-(3-(4-cyanphenyl)propionsäurepiperidid)-pyrrolidin-2-on

Das gemäß f) erhaltene Produkt wurde in 25 m1 Methylenchlorid gelöst und mit 5,67 g (19,8 mmol) 6,7-Dimethoxynaphthalin-2-sulfonsäurechlorid, gelöst ist 25 ml Methylenchlorid, versetzt. Anschließend gab man 7,6 ml Triethylamin zu und rührte über Nacht bei Raumtemperatur. Das Reaktionsgemisch wurde im Vakuum eingeengt, in Essigester aufgenommen, mit 2N Salzsäure und anschließend mit gesättigter Kochsalzlösung extrahiert, über Magnesiumsulfat getrocknet und eingeengt. Das Gemisch wurde säulenchromatographisch gereinigt (250 ml Kieselgel 0,063-0,200 mm, Methylenchlorid mit 0-3 % Methanol ansteigend). Man erhielt 7,75 g fast reines 3-(6,7-Dimethoxy-naphthalin-2-sulfonylamino)-1-(2-(3-(4-cyanphenyl)propionsäurepiperidid)-pyrrolidin-2-on (Ausbeute über zwei Stufen 66 %).

h) Darstellung von 3-(6,7-Dimethoxy-naphthalin-2-sulfonylamino)-1-(2-(3-(4-aminothiocarbonylphenyl)propionsäure-piperidid))-pyrrolidin-2-on

4,6 g (7,78 mmol) 3-(6,7-Dimethoxy-naphthalin-2-sulfonylamino)-1(2(3(4-cyanphenyl)propionsäurepiperidid))-pyrrolidin-2-on wurden in 5 ml Triethylamin und 80 ml Pyridin gelöst und Schwefelwasserstoff bis zur Sättigung eingeleitet. Die Reaktionsmischung ließ man über Nacht stehen und tropfte sie anschließend in ein Gemisch aus 600 g Eis und 100 ml konzentrierter Salzsäure. Der Niederschlag wurde abgesaugt, in THF gelöst, über Magnesiumsulfat getrocknet und eingeengt. Der Rückstand wurde mit Ether versetzt, 30 min bei Raumtemperatur gerührt und der Niederschlag abgesaugt. Das leicht verunreinigte 3-(6,7-Dimethoxy-naphthalin-2-sulfonylamino)-1-[2-(3-(4-aminothiocarbonylphenyl)-propionsäurepiperidid)]-pyrrolidin-2-on wurde ohne weitere Reinigung in die nächste Stufe eingesetzt.

i) Darstellung von 3-(6,7-Dimethoxy-naphthalin-2-sulfonylamino) -1-[2-(3-(4-(methylthio-iminomethyl) -phenyl)-propionsäurepiperidid)]-pyrrolidin-2-on

Das gemäß h) erhaltene Rohprodukt wurde mit 10 ml Methyljodid versetzt und 75 min bei Raumtemperatur gerührt. Anschließend wurde zunächst im Wasserstrahlvakuum, dann im Hochvakuum bei Raumtemperatur zur Trokkene eingeengt. Das mäßig verunreinigte 3-(6,7-Dimethoxy-naphthalin-2-sulfonylamino)-1-[2-(3-(4-(methylthioiminomethyl)-phenyl)-propionsäurepiperidid)]-pyrrolidin-2-on wurde ohne Reinigung in die nächste Stufe eingesetzt.

j) Darstellung von 3-(6,7-Dimethoxy-naphthalin-2-sulfonylamino)-1-[2-(3-(4-amidinophenyl)-propionsäurepiperidid)]-pyrrolidin-2-on Hydrojodid

Das gemäß i) erhaltene Rohprodukt wurde zusammen mit 1,28 g Ammoniumacetat und 80 ml Methanol 90 min bei 60-65°C gerührt und anschließend im Vakuum eingeengt. Der Rückstand wurde in 80 ml Methylenchlorid aufgenommen und unlösliche Anteile wurden abfiltriert. Das Filtrat wurde im Vakuum eingeengt. Der Rückstand wurde in wenig Ethanol aufgenommen und mit Essigester das Produkt ausgefällt. Diese Reinigungsprozedur wurde mehrfach wiederholt. Die gesammelten Mutterlaugen ließ sich ebenfalls auf diese Weise aufreinigen. Man erhielt 2,9 g (≙ 50,6 % über 3 Stufen) reines 3-(6,7-Dimethoxy-naphthalin-2-sulfonylamino)-1-[2-(3-(4-amidinophenyl)-propionsäurepiperidid)]-pyrrolidin-2-on als Hydrojodid als weiße Festsubstanz.

Das Diastereomerengemisch von (3S)-3-(6,7-Dimethoxynaphthalin-2-sulfonylamino)-1-[2(R,S)-2-(3-(4-amidinophenyl)-propionsäurepiperidid)]-pyrrolidin-2-on ließ sich mittels MPLC trennen.

Das für die Umsetzung g) benötigte 6,7-Dimethoxy-naphthalin-2-sulfonsäurechlorid wurde wie folgt hergestellt:
9,6 g 50 %ige Natriumhydridsuspension (200 mmol) wurden in Hexan vorgelegt, entölt und mit ca. 250 ml DMF

versetzt. Dazu tropfte man bei Raumtemperatur die Lösung von 14,4 g 6,7-Dihydroxy-naphthalin-2-natriumsulfonat (55 mmol) in 200 ml DMF und ließ nach beendeter Wasserstoffentwicklung noch 45 min nachreagieren. Anschließend tropfte man innerhalb von 20 min 51,1 g (360 mmol) Methyljodid zu, wobei die Temperatur 25°C nicht überstieg, und rührte über Nacht bei Raumtemperatur. Anschließend wurde die Reaktionsmischung in wenig Eiswasser eingetragen, danach im Vakuum eingeengt und mehrfach in Aceton aufgenommen und eingeengt. Der Rückstand wurde aus 1 1 Aceton umkristallisiert.

Das so erhaltene Rohprodukt von 6,7-Dimethoxy-naphthalin-2-natriumsulfonat, welches noch etwas anorganisches Salz enthielt, wurde ohne weitere Reinigung zusammen mit 45 ml Thionylchlorid 50 min bei 60°C gerührt, anschließend im Vakuum eingeengt und der Rest in Methylenchlorid aufgenommen. Unlösliche Bestandteile wurden abfiltriert, die Methylenchloridlösung gewaschen, über Magnesiumsulfat getrocknet und im Vakuum eingeengt. Der Rückstand wurde in 1 1 Ether aufgenommen, mit Aktivkohle versetzt, abfiltriert und auf 100 ml eingeengt. Beim Abkühlen kristallisierte das Produkt aus, welches abgesaugt und mit kaltem Ether nachgewaschen wurde.

Man erhielt 9,1 g (≙ 58 %) reines 6,7-Dimethoxy-naphthalin-2-sulfonsäurechlorid.

Beispiel 2

a) Darstellung von N-(Naphthalin-2-sulfonyl)-L- bzw. D-glutaminsäuredimethylester

28,4 g (134,2 mmol) L- oder D-Glutaminsäuredimethylesterhydrochlorid wurden in 300 ml Methylenchlorid vorgelegt. Dazu wurden nacheinander 70 ml (51,2 g, 505 mmol) Triethylamin und 25,8 g (113,8 mmol) Naphthalin-2-sulfonsäurechlorid - gelöst in 100 ml THF - bei 10 - 15°C zugetropft. Danach wurde 1 h bei Raumtemperatur gerührt, anschließend die Reaktionslösung im Vakuum eingeengt, der Rückstand in Ether/Essigester aufgenommen, zunächst mit verdünnter wäßriger Amidosulfonsäurelösung, dann mehrfach mit Wasser gewaschen, die organische Phase über Magnesiumsulfat getrocknet und im Vakuum eingeengt. Man erhielt 33,4 g (76 % d.Th.) reinen N-(Naphthalin-2-sulfonyl)-L- bzw. D-glutaminsäuredimethylester.

b) Darstellung von N-(Naphthalin-2-sulfonyl)-L- bzw. D-glutaminsäure

29,5 g (80,7 mmol) N-(Naphthalin-2-sulfonyl)-L- oder D-glutaminsäuredimethylester wurden in 500 ml Methanol gelöst und mit 170 ml (340 mmol) 2N Natronlauge über Nacht gerührt. Mit konzentrierter Salzsäure wurde auf pH 1 gestellt, im Vakuum die Reaktionslösung zur Trockene eingeengt, der Rückstand in viel Methylenchlorid /wenig Wasser verteilt, die Methylenchloridphase über Magnesiumsulfat getrocknet, einrotiert und der verbliebene Rückstand aus Methylenchlorid umkristallisiert. Dabei erhielt man 20,3 g (75 % d.Th.) N-(Naphthalin-2-sulfonyl)-L- bzw. D-glutaminsäure als weiße Festsubstanz.

c) Darstellung von 3-[3-(Naphthalin-2-sulfonylamino)-oxazolidin-5-on-4-yl]-propionsäure

19,3 g (57,2 mmol) N-(Naphthalin-2-sulfonyl)-glutaminsäure wurden zusammen mit 3,4 g (114,4 mmol) Paraformaldehyd, 0,5 g (4 mmol) Thionylchlorid und 6,1 g (60 mmol) Acetanhydrid in 140 ml Eisessig 5 h auf 100°C erhitzt. Aufgrund unvollständiger Reaktion wurden weitere 3,4 g (114,4 mmol) Paraformaldehyd, 0,5 g (4 mmol) Thionylchlorid und 6,1 g (60 mmol) Acetanhydrid zugegeben und 8 h auf 100°C erhitzt. Nach Entfernen des Lösungsmittels im Vakuum wurde der Rückstand in Methylenchlorid aufgenommen, mit Wasser gewaschen, über Magnesiumsulfat getrocknet und im Vakuum eingeengt. Zur Entfernung von Essigsäure wurde der Feststoff mehrfach mit Wasser gewaschen. Nach dem Trocknen wurde das Produkt mit Diisopropylether ausgekocht, wonach man durch Einrotieren des Filtrats 15,3 g (76 % d.Th.) fast reine 3-[3-(Naphthalin-2-sulfonylamino)-oxazolidin-5-on-4-yl]-propionsäure erhielt.

d) Darstellung von 3-[3-(Naphthalin-2-sulfonylamino)-oxazolidin-5-on-4-yl]-propanal

1,4 ml (18,2 mmol) trockenes Dimethylformamid wurden in 50 ml Methylenchlorid vorgelegt. Bei 0°C wurden unter Inertgasatmosphäre 2,0 ml (28,2 mmol) Oxalylchlorid in 70 ml Methylenchlorid zugetropft, 30 min bei 0°C gerührt und anschließend das Lösungsmittel im Vakuum entfernt. Das zurückbleibende weiße Salz (stark hygroskopisch!) wurde in 120 ml THF/Acetonitril (1:1) suspendiert und auf -30°C abgekühlt. Dazu tropfte man 5,6 g (16 mmol) 3-[3-(Naphthalin-2-sulfonylamino)-oxazolidin-5-on-4-yl]-propionsäure zusammen mit 1,07 ml (16 mmol) Pyridin in 30 ml THF und rührte anschließend 20 min bei -20°C weiter. Nachdem das Reaktionsgemisch auf -75°C abgekühlt und 307 mg Cu-I-jodid zugegeben worden war, wurde eine Lösung von 5,6 g (21,8 mmol) Lithiumtritert.-butoxyaluminiumhydrid in 20 ml THF langsam zugetropft und danach 1 h bei dieser Temperatur gerührt. Anschließend wurde mit 30 ml 2N Salzsäure-Lösung bei -75°C gequencht, nach Erwärmen auf Raumtemperatur das Lösungsmittel im Vakuum entfernt, der Rückstand in Methylenchlorid aufgenommen, die Cu-Salze abfiltriert, die organische Phase dreimal mit Wasser gewaschen, über Magnesiumsulfat getrocknet und im Vakuum eingeengt. Man erhielt 5,4 g wenig verunreinigtes 3-[3-(Naphthalin-2-sulfonylamino)-oxazolidin-5-on-4-yl]-propanal, welches wegen seiner mäßigen Stabilität rasch weiterverarbeitet wurde.

e) Darstellung von 3-(Naphthalin-2-sulfonylamino)-1-[2-(3-(4-cyanphenyl)-propionsäurepiperidid)-piperidin-2-on

Zu 7,5 g frisch aktiviertem Molekularsieb (4 Å) in 30 ml Methanol wurden nacheinander 2,5 g (~ 7,5 mmol) 3-[3-(Naphthalin-2-sulfonylamino)-oxazolidin-5-on-4-yl]-propanal-Rohprodukt in 15 ml Methylenchlorid, 2,2 g (7,5 mmol) p-Cyanphenylalaninylpiperidid-hydrochlorid in 15 ml Methylenchlorid und 1,2 g (14,6 mmol) Natriumacetat gegeben. Nachdem auf ca. 12°C abgekühlt worden war, tropfte man 0,9 g (14,3 mmol) Natriumcyanborhydrid - gelöst in 20 ml THF - in 35 min zu und rührte 3 h bei Raumtemperatur. Die festen Bestandteile wurden anschließend abfiltriert, mit Methylenchlorid nachgewaschen, die gesammelten Filtrate im Vakuum einrotiert, der Rückstand in Essigester aufgenommen, mit verdünnter wäßriger Amidosulfonsäurelösung (pH 1) und fünfmal mit gesättigter Kochsalzlösung gewaschen, über Magnesiumsulfat getrocknet und im Vakuum einrotiert. Nach Chromatographie über Kieselgel (0,063-0,200 mm/Eluent Methylenchlorid/Methanol) erhielt man 3,5 g (85 % d.Th. über 3 Stufen) reines 3-(Naphthalin-2-sulfonylamino)-1-[2-(3-(4-cyanphenyl)-propionsäurepiperidid)] -piperidin-2-on.

f) Die anschließenden Reaktionen zur Darstellung von 3-(Naphthalin-2-sulfonylamino)-1-[2-(3-(4-amidinophenyl)-propionsäurepiperidid)]-piperidin-2-on erfolgten in Analogie zu 1h, i und j.

Beispiel 3

a) Darstellung von 2-(3-Nitropyrid-2-on-1-yl)-essigsäure-tert.-butylester

63,3 g (459 mmol) pulverisiertes Kaliumcarbonat wurden in 100 ml DMF suspendiert, auf 0°C abgekühlt und bei dieser Temperatur mit einer Suspension von 21,5 g (153 mmol) 2-Hydroxy-3-nitro-pyridin in 100 ml DMF versetzt. Nach 10 min Rühren bei 0°C wurden 23,6 ml (31,4 g, 161 mmol) Bromessigsäure-tert.-butylester in 50 ml DMF zugetropft und anschließend 45 min weitergerührt, wobei eine intensive Rotfärbung auftrat. Kaliumcarbonat wurde abgesaugt, das Filtrat mit wäßriger Salzsäure sauer gestellt (pH 3), mit Methylenchlorid extrahiert, die organische Phase zweimal mit Wasser gewaschen, über Magnesiumsulfat getrocknet und eingedampft. Nachdem noch anhaftendes DMF und Bromessigsäure-tert.-butylester im Hochvakuum entfernt worden waren, wurde das Produkt mit Diisopropylether ausgerührt, wobei man 35,5 g (90 % d.Th.) 2-(3-Nitropyrid-2-on-1-yl)-essigsäure-tert.-butylester erhielt.

b) Darstellung von 2- (3-Nitropyrid-2-on-1-yl)-essigsäure

35,5 g (140 mmol) 2-(3-Nitropyrid-2-on-1-yl)-essigsäure-tert.-butylester in 110 ml Methylenchlorid wurden mit 107 ml (159,0 g, 1,40 mol) Trifluoressigsäure versetzt und 2 h bei Raumtemperatur gerührt. Das Reaktionsgemisch wurde im Vakuum eingeengt, der Rückstand mit Diisopropylether verrührt und der Feststoff abgesaugt. Dabei erhielt man 22,1 g (80 % d.Th.) 2-(3-Nitropyrid-2-on-1-yl)-essigsäure.

c) Darstellung von 2-(3-Nitropyrid-2-on-1-yl)-essigsäure-piperidid

10 g (50,5 mmol) 2-(3-Nitropyrid-2-on-1-yl)-essigsäure wurden in 500 ml THF gelöst, auf -20°C abgekühlt, tropfenweise mit 7 ml (6,7 g, 55,6 mmol) Pivalinsäurechlorid in 30 ml THF und 7 ml (5,1 g, 50,5 mmol) Triethylamin in ebenfalls 30 ml THF versetzt und danach noch 20 min bei -20°C gerührt. Anschließend wurden 5 ml (4,3 g, 50,5 mmol) Piperidin in 40 ml THF zugetropft und das Reaktionsgemisch weitere 30 min bei -20°C gerührt. Die Reaktionslösung wurde mit 1N Salzsäure sauer gestellt, mehrfach mit Methylenchlorid extrahiert, die organische Phase mit Wasser gewaschen, über Magnesiumsulfat getrocknet und im Vakuum eingeengt. Der Rückstand wurde mehrmals mit Hexan ausgekocht und heiß filtriert. Man erhielt dabei 11,8 g (88 % d.Th.) 2-(3-Nitropyrid-2-on-1-yl)-essigsäurepiperidid, welches noch geringe Verunreinigungen an Pivaloylpiperidid enthielt.

d) Darstellung von 2-(3-Aminopyrid-2-on-1-yl)-essigsäure-piperidid-hydrochlorid

22,8 g (86 mmol) 2-(3-Nitropyrid-2-on-1-yl)-essigsäure-piperidid wurden in 460 ml Methanol gelöst, mit 9,8 ml (10,3 g, 172 mmol) Eisessig und 3,4 g 10 % Palladium auf Aktivkohle versetzt und mit Wasserstoff bei leichtem Überdruck hydriert. Nach Reaktionsende wurde vom Katalysator abgesaugt, letzterer mit Methanol gewaschen und die Filtrate im Vakuum eingeengt. Der Rückstand wurde in wäßriger Amidosulfonsäurelösung aufgenommen, zweimal mit Methylenchlorid extrahiert (enthielt hauptsächlich Pivaloylpiperidid), die wäßrige Phase alkalisiert, viermal mit Methylenchlorid extrahiert, die Extrakte über Magnesiumsulfat getrocknet, mit etherischer Salzsäure sauer gestellt und im Vakuum eingeengt. Man erhielt 14,2 g (61 % d.Th.) 2-(3-Aminopyrid-2-on-1-yl)-essigsäure-piperidid-hydrochlorid.

e) Darstellung von 2- [3- (Naphthalin-2-sulfonylamino)-pyrid-2-on-1-yl]-essigsäurepiperidid.

Zu einer Lösung von 14,2 g (53,5 mmol) 2-(3-Aminopyrid-2-on-1-yl)-essigsäurepiperidid-hydrochlorid und 43,2 ml (42,3 g, 535 mmol) Pyridin in 250 ml Methylenchlorid tropfte man 10,3 g (45,5 mmol) Naphthalin-2-sulfonsäurechlorid in 100 ml Methylenchlorid und rührte 1,5 h bei Raumtemperatur. Anschließend wurde die Reaktionslösung

mit 1N Salzsäure sauer gestellt, die organische Phase einmal mit Wasser gewaschen, über Magnesiumsulfat getrocknet und im Vakuum eingeengt. Man erhielt 18,4 g (95 % d.Th.) 2-[3-(Naphthalin-2-sulfonylamino)-pyrid-2-on-1-yl]-essigsäurepiperidid.

f) Darstellung von 2- [3-(Naphthalin-2-sulfonylamino-N-tert.-butyloxycarbonyl)-pyrid-2-on-1-yl]-essigsäurepiperid

18,4 g (43 mmol) 2-[3-(Naphthyl-2-sulfonylamino-pyrid-2-on-1-yl]-essigsäurepiperid wurden in 300 ml Methylenchlorid gelöst und nacheinander mit 9,4 g (43 mmol) Di-tert.-butylcarbonat und 5,3 g (43 mmol) Dimethylaminopyridin versetzt, wobei eine homogene Lösung entstand, die noch 40 min weitergerührt wurde. Anschließend wurde die Reaktionslösung mit 1N Salzsäure auf pH 2,5 gestellt, die organische Phase einmal mit Wasser gewaschen, über Magnesiumsulfat getrocknet und im Vakuum einrotiert. Nach Ausrühren aus Hexan erhielt man 22,0 g (97,3 % d.Th.) 2-[3-(Naphthalin-2-sulfonylamino-N-tert.-butyloxycarbonyl)-pyrid-2-on-1-yl]-essigsäure-piperidid.

g) Darstellung von 3- (Naphthalin-2-sulfonylamino-N-tert.-butyloxycarbonyl)-1-(2-(3-(4-cyanphenyl)-propionsäure-piperidid))-pyrid-2-on

Zu 1,4 ml (1,03 g, 10,14 mmol) Diisopropylamin in 40 ml THF wurden bei -70°C nacheinander tropfenweise 6,1 ml (10,14 mmol) 15 %ige Butyllithiumlösung in Hexan, 4,1 g (7,8 mmol) 2-[3-(Naphthalin-2-sulfonylamino-N-tert.-butyloxycarbonyl)-pyrid-2-on-1-yl]-essigsäurepiperidid in 40 ml THF und 1,4 g (7,02 mmol) p-Cyanbenzylbromid in 30 ml THF gegeben. Nach 2 h Rühren bei -70°C ließ man den Ansatz auf Raumtemperatur erwärmen, gab Methylenchlorid zu, stellte mit 1N Salzsäure auf pH 2,5 und extrahierte die wäßrige Phase. Die organische Phase wurde mit Wasser gewaschen, mit Magnesiumsulfat getrocknet und im Vakuum eingeengt. Das entstandene Rohprodukt wurde säulenchromatographisch gereinigt (Kieselgel 0,063-0,200 mm, Eluent: Methylenchlorid/Methanol). Man erhielt 2,5 g (56 % d.Th.) 3-(Naphthalin-2-sulfonylamino-N-tert.-butyloxycarbonyl)-1-(2-(3-(4-cyanphenyl)-propionsäurepiperidid))-pyrid-2-on.

h) Die Darstellung von 3-(Naphthalin-2-sulfonylamino-N-tert.-butyloxycarbonyl)-1-(2-(3-(4-amidinophenyl)-propionsäurepiperidid))-pyrid-2-on wurde in Analogie zu 1h, i und j durchgeführt. Nach Abspaltung der BOC-Schutzgruppe (Trifluoressigsäure/Methylenchlorid) wurde das 3-(Naphthalin-2-sulfonylamino)-1-(2-(3-(4-amidinophenyl)-propionsäurepiperidid))-pyrid-2-on als Trifluoracetat erhalten.

Analog den Beispielen 1 bis 3 lassen sich darstellen:

4) 3-(Naphthalin-2-sulfonylamino)-1-[2-(3-(4-amidinophenyl)-propionsäurepiperidid)]-pyrrolidin-2-on

5) 3-(Naphthalin-2-sulfonylamino) -1-[2-(3-(4-amidinophenyl)-propionsäureethylester)]-piperidin-2-on

6) 3-(Naphthalin-2-sulfonylamino)-1-[2-(3-(4-amidinophenyl)-propionsäure)]-piperidin-2-on

7) 3-(Naphthalin-2-sulfonylamino) -1-[2-(3-(4-amidinophenyl)-propionsäureethylester)]-pyrrolidin-2-on

8) 3-(Naphthalin-2-sulfonylamino)-1-[2-(3-(4-amidinophenyl)-propionsäure)]-pyrrolidin-2-on

9) 3-(Naphthalin-2-sulfonylamino)-1-[2-(3-(4-amidinophenyl)-propionsäureethylester)]-pyrid-2-on

10) 3-(Naphthalin-2-sulfonylamino)-1-[2-(3-(4-amidinophenyl)-propionsäure)]-pyrid-2-on

11) 3-(4-Methoxynaphthalin-2-sulfonylamino)-1-[2-(3-(4-amidinophenyl)-propionsäurepiperidid)]-piperidin-2-on

12) 3-(4-Methoxynaphthalin-2-sulfonylamino)-1-[2-(3-(4-amidinophenyl)-propionsäureethylester)]-piperidin-2-on

13) 3-(4-Methoxynaphthalin-2-sulfonylamino)-1-[2-(3-(4-amidinophenyl)-propionsäure)]-piperidin-2-on

14) 3-(4-Methoxynaphthalin-2-sulfonylamino)-1-[2-(3-(4-amidinophenyl)-propionsäurepiperidid)]-pyrrolidin-2-on

15) 3-(4-Methoxynaphthalin-2-sulfonylamino)-1-[2-(3-(4-amidinophenyl)-propionsäureethylester)]-pyrrolidin-2-on

16) 3-(4-Methoxynaphthalin-2-sulfonylamino)-1-[2-(3-(4-amidinophenyl)-propionsäure)]-pyrrolidin-2-on

17) 3-(4-Methoxynaphthalin-2-sulfonylamino)-1-[2-(3-(4-amidinophenyl)-propionsäurepiperidid)]-piperid-2-on

18) 3-(4-Methoxynaphthalin-2-sulfonylamino)-1-[2-(3-(4-amidinophenyl)-propionsäureethylester)]-piperid-2-on

19) 3-(4-Methoxynaphthalin-2-sulfonylamino)-1-[2-(3-(4-amidinophenyl)-propionsäure)]-piperid-2-on

20) 3-(6-Methoxynaphthalin-2-sulfonylamino)-1-[2-(3-(4-amidinophenyl)-propionsäurepiperidid)]-piperidin-2-on

21) 3-(6-Methoxynaphthalin-2-sulfonylamino)-1-[2-(3-(4-amidinophenyl)-propionsäureethylester)]-piperidin-2-on

22) 3-(6-Methoxynaphthalin-2-sulfonylamino)-1-[2-(3-(4-amidinophenyl)-propionsäure)]-piperidin-2-on

23) 3-(6-Methoxynaphthalin-2-sulfonylamino)-1-[2-(3-(4-amidinophenyl)-propionsäurepiperidid)]-pyrrolidin-2-on

24) 3-(6-Methoxynaphthalin-2-sulfonylamino)-1-[2-(3-(4-amidinophenyl)-propionsäureethylester)]-pyrrolidin-2-on

25) 3-(6-Methoxynaphthalin-2-sulfonylamino)-1-[2-(3-(4-amidinophenyl)-propionsäure)]-pyrrolidin-2-on

26) 3-(6-Methoxynaphthalin-2-sulfonylamino)-1-[2-(3-(4-amidinophenyl)-propionsäurepiperidid)]-piperid-2-on

27) 3-(6-Methoxynaphthalin-2-sulfonylamino)-1-[2-(3-(4-amidinophenyl)-propionsäureethylester)]-piperid-2-on

28) 3-(6-Methoxynaphthalin-2-sulfonylamino)-1-[2-(3-(4-amidinophenyl)-propionsäure)]-piperid-2-on

29) 3-(7-Methoxynaphthalin-2-sulfonylamino)-1-[2-(3-(4-amidinophenyl)-propionsäurepiperidid)]-piperidin-2-on

30) 3-(7-Methoxynaphthalin-2-sulfonylamino)-1-[2-(3-(4-amidinophenyl)-propionsäureethylester)]-piperidin-2-on

31) 3-(7-Methoxynaphthalin-2-sulfonylamino)-1-[2-(3-(4-amidinophenyl)-propionsäure)]-piperidin-2-on

32) 3-(7-Methoxynaphthalin-2-sulfonylamino)-1-[2-(3-(4-amidinophenyl)-propionsäurepiperidid)]-pyrrolidin-2-on

33) 3-(7-Methoxynaphthalin-2-sulfonylamino)-1-[2-(3-(4-amidinophenyl)-propionsäureethylester)]-pyrrolidin-2-on

34) 3-(7-Methoxynaphthalin-2-sulfonylamino)-1-[2-(3-(4-amidinophenyl)-propionsäure)]-pyrrolidin-2-on

35) 3-(7-Methoxynaphthalin-2-sulfonylamino)-1-[2-(3-(4-amidinophenyl)-propionsäurepiperidid)]-piperid-2-on

36) 3-(7-Methoxynaphthalin-2-sulfonylamino)-1-[2-(3-(4-amidinophenyl)-propionsäureethylester)]-piperid-2-on

37) 3-(7-Methoxynaphthalin-2-sulfonylamino)-1-[2-(3-(4-amidinophenyl)-propionsäure)]-piperid-2-on

38) 3-(6,7-Dimethoxynaphthalin-2-sulfonylamino)-1-[2-(3-(4-amidinophenyl)-propionsäurepiperidid)]-piperidin-2-on

39) 3-(6,7-Dimethoxynaphthalin-2-sulfonylamino)-1-[2-(3-(4-amidinophenyl)-propionsäureethylester)]-piperidin-2-on

40) 3-(6,7-Dimethoxynaphthalin-2-sulfonylamino)-1-[2-(3-(4-amidinophenyl)-propionsäure)]-piperidin-2-on

41) 3-(6,7-Dimethoxynaphthalin-2-sulfonylamino)-1-[2-(3-(4-amidinophenyl)-propionsäurepiperidid)]-pyrrolidin-2-on

42) 3-(6,7-Dimethoxynaphthalin-2-sulfonylamino)-1-[2-(3-(4-amidinophenyl)-propionsäureethylester)]-pyrrolidin-2-on

43) 3-(6,7-Dimethoxynaphthalin-2-sulfonylamino)-1-[2-(3-(4-amidinophenyl)-propionsäure)]-pyrrolidin-2-on

44) 3-(6,7-Dimethoxynaphthalin-2-sulfonylamino)-1-[2-(3-(4-amidinophenyl)-propionsäurepiperidid)]-piperid-2-on

45) 3-(6,7-Dimethoxynaphthalin-2-sulfonylamino)-1-[2-(3-(4-amidinophenyl)-propionsäureethylester)]-piperid-2-on

46) 3-(6,7-Dimethoxynaphthalin-2-sulfonylamino)-1-[2-(3-(4-amidinophenyl)-propionsäure)]-piperid-2-on

47) 3-(4,6-Dimethoxynaphthalin-2-sulfonylamino)-1-[2-(3-(4-amidinophenyl)-propionsäurepiperidid)]-piperidin-2-on

48) 3-(4,6-Dimethoxynaphthalin-2-sulfonylamino)-1-[2-(3-(4-amidinophenyl)-propionsäureethylester)]-piperidin-2-on

49) 3-(4,6-Dimethoxynaphthalin-2-sulfonylamino)-1-[2-(3-(4-amidinophenyl)-propionsäure)]-piperidin-2-on

50) 3-(4,6-Dimethoxynaphthalin-2-sulfonylamino)-1-[2-(3-(4-amidinophenyl)-propionsäurepiperidid)]-pyrrolidin-2-on

51) 3-(4,6-Dimethoxynaphthalin-2-sulfonylamino)-1-[2-(3-(4-amidinophenyl)-propionsäureethylester)]-pyrrolidin-2-on

52) 3-(4,6-Dimethoxynaphthalin-2-sulfonylamino)-1-[2-(3-(4-amidinophenyl)-propionsäure)]-pyrrolidin-2-on

53) 3-(4,6-Dimethoxynaphthalin-2-sulfonylamino)-1-[2-(3-(4-amidinophenyl)-propionsäurepiperidid)]-piperid-2-on

54) 3-(4,6-Dimethoxynaphthalin-2-sulfonylamino)-1-[2-(3-(4-amidinophenyl)-propionsäureethylester)]-piperid-2-on

55) 3-(4,6-Dimethoxynaphthalin-2-sulfonylamino)-1-[2-(3-(4-amidinophenyl)-propionsäure)]-piperid-2-on

56) 3-(6,7-Dimethoxynaphthalin-2-sulfonylamino)-1-[2-(3-(4-amidinophenyl)-propionsäurepyrrolidid)]-piperidin-2-on

57) 3-(6,7-Dimethoxynaphthalin-2-sulfonylamino)-1-[2-(3-(4-amidinophenyl)-propionsäuremorpholid)]-piperidin-2-on

58) 3-(6,7-Dimethoxynaphthalin-2-sulfonylamino)-1-[2-(3-(4-amidinophenyl)-propionsäurehexahydroazepid)]-piperidin-2-on

59) 3-(6,7-Dimethoxynaphthalin-2-sulfonylamino)-1-[2-(3-(4-amidinophenyl)-propionsäurepyrrolidid)]-pyrrolidin-2-on

60) 3-(6,7-Dimethoxynaphthalin-2-sulfonylamino)-1-[2-(3-(4-amidinophenyl)-propionsäuremorpholid)]-pyrrolidin-2-on

61) 3-(6,7-Dimethoxynaphthalin-2-sulfonylamino)-1-[2-(3-(4-amidinophenyl)-propionsäurehexahydroazepid)]-pyrrolidin-2-on

62) 3-(6,7-Methylendioxynaphthalin-2-sulfonylamino)-1-[2-(3-(4-amidinophenyl)-propionsäurepiperidid)]-piperidin-2-on

63) 3-(6,7-Methylendioxynaphthalin-2-sulfonylamino)-1-[2-(3-(4-amidinophenyl)-propionsäureethylester)]-piperidin-2-on

64) 3-(6,7-Methylendioxynaphthalin-2-sulfonylamino)-1-[2-(3-(4-amidinophenyl)-propionsäure)]-piperidin-2-on

65) 3-(6,7-Methylendioxynaphthalin-2-sulfonylamino)-1-[2-(3-(4-amidinophenyl)-propionsäurepiperidid)]-pyrrolidin-2-on

66) 3-(6,7-Methylendioxynaphthalin-2-sulfonylamino)-1-[2-(3-(4-amidinophenyl)-propionsäureethylester)]-pyrrolidin-2-on

67) 3-(6,7-Methylendioxynaphthalin-2-sulfonylamino)-1-[2-(3-(4-amidinophenyl)-propionsäure)]-pyrrolidin-2-on

68) 3-(6,7-Ethylendioxynaphthalin-2-sulfonylamino)-1-[2-(3-(4-amidinophenyl)-propionsäurepiperidid)]-piperidin-2-on

69) 3-(6,7-Ethylendioxynaphthalin-2-sulfonylamino)-1-[2-(3-(4-amidinophenyl)-propionsäurepiperidid)]-pyrrolidin-2-on

## Patentansprüche

**1.** 2-[3-(4-Amidino-phenyl)]-propionsäurederivate der Formel

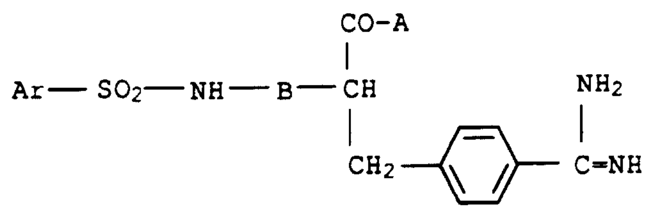

worin

A

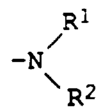

oder $-OR^3$ bedeutet, worin $R^1$ und $R^2$, die gleich oder verschieden sein können, für wasserstoff, gesättigte oder ungesättigte Alkylreste mit bis zu 6 C-Atomen oder Aralkylreste oder Arylreste stehen, oder $R^1$ und $R^2$ zusammen mit dem sie verbindenden Stickstoffatom einen 5- bis 7-gliedrigen gesättigten Ring bedeuten, der gegebenenfalls durch Alkylreste mit bis zu 4 C-Atomen substituiert ist und der ein Sauerstoffatom enthalten kann, wobei die Stickstoff- und Sauerstoffatome in 1,2- bzw. 1,4-Stellung stehen,
und worin $R^3$ für Wasserstoff, einen gesättigten oder ungesättigten Alkylrest mit bis zu 6 C-Atomen, eine Cycloalkylgruppe oder eine Aralkylgruppe steht,

B

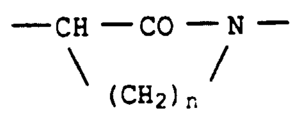

(mit n in der Bedeutung 1, 2, 3, 4 oder 5) oder die Gruppe

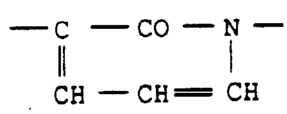

darstellt,

Ar eine Phenyl- oder α-, oder β-Naphthylgruppe bedeutet, die gegebenenfalls durch ein oder mehrere Halogenatome, Nitrogruppen, Aminogruppen, $C_{1-4}$-Mono- bzw. Bisalkylaminogruppen, Hydroxygruppen, $C_{1-4}$-Alkylreste, $C_{1-4}$-Alkoxygruppen, einen Methylendioxy- oder Ethylendioxyrest substituiert ist, oder Ar eine Pyridyl-,

Chinolyl- oder Isochinolylgruppe bedeutet, die gegebenenfalls durch eine oder mehrere $C_{1-4}$-Alkylqruppen oder $C_{1-4}$-Alkoxygruppen substituiert sind,

sowie deren Salze mit physiologisch verträglichen Säuren.

2. Verfahren zur Herstellung der Verbindungen der Formel I gemäß Anspruch 1, dadurch gekennzeichnet, daß man eine Verbindung der Formel II,

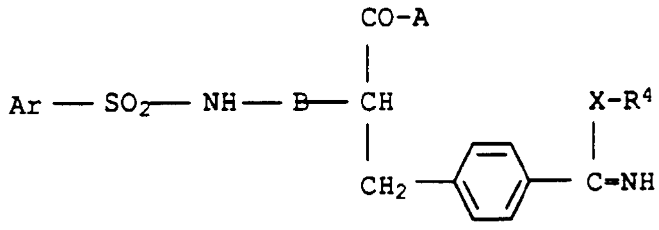

II,

worin $R^4$ $C_{1-3}$-Alkyl oder Benzyl und X ein Sauerstoff- oder Schwefelatom bedeuten, mit Ammoniak oder einem Ammoniumsalz umsetzt und die so erhaltenen Verbindungen gegebenenfalls in ihre Salze mit physiologisch verträglichen Säuren überführt.

3. Verbindungen der Formel I gemäß Anspruch 1 zur Verwendung bei der Bekämpfung von Krankheiten.

**Claims**

1. A 2-[3-(4-amidinophenyl)]propionic acid derivative of the formula

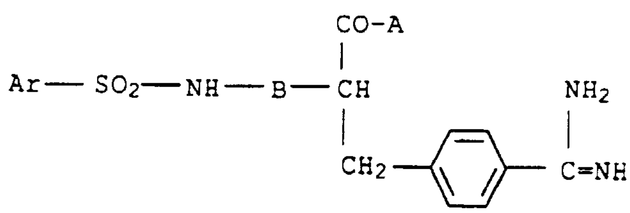

where

A is

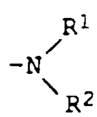

or $-OR^3$ in which $R^1$ and $R^2$, which can be identical or different, are each hydrogen, saturated or unsaturated alkyl with up to 6 carbons or aralkyl or aryl, or $R^1$ and $R^2$ together with the nitrogen to which they are bonded are a 5- to 7-membered saturated ring which is unsubstituted or substituted by alkyl radicals with up to 4 carbons and can contain an oxygen atom, where the nitrogen and oxygen atoms are in the 1,2 or 1,4 positions, and where $R^3$ is hydrogen, saturated or unsaturated alkyl with up to 6 carbons, cycloalkyl or aralkyl,

B is

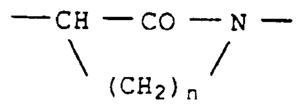

(where n is 1, 2, 3, 4 or 5) or

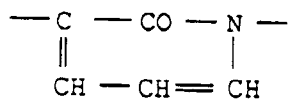

Ar is phenyl or α- or β-naphthyl which is unsubstituted or substituted by one or more halogen atoms, nitro groups, amino groups, $C_{1-4}$-mono- or bisalkylamino groups, hydroxyl groups, $C_{1-4}$-alkyl radicals, $C_{1-4}$-alkoxy groups, a methylenedioxy or ethylenedioxy radical, or Ar is pyridyl, quinolyl or isoquinolyl which is unsubstituted or substituted by one or more $C_{1-4}$-alkyl groups or $C_{1-4}$-alkoxy groups,

and the salts thereof with physiologically tolerated acids.

**2.** A process for preparing a compound of the formula I as claimed in claim 1, which comprises reacting a compound of the formula II

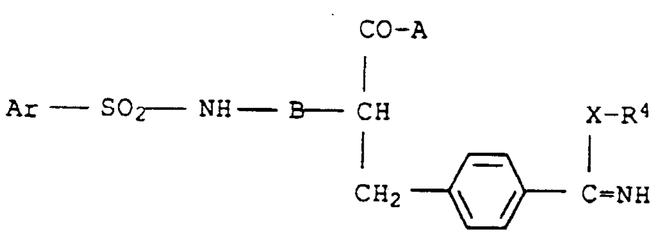

II,

where $R^4$ is $C_{1-3}$-alkyl or benzyl and X is oxygen or sulfur, with ammonia or an ammonium salt, and converting the resulting compounds where appropriate into their salts with physiologically tolerated acids.

**3.** A compound of the formula I as claimed in claim 1 for use for controlling diseases.

**Revendications**

**1.** Dérivés de l'acide 2-[3-(4-amidinophényl)]-propionique de formule

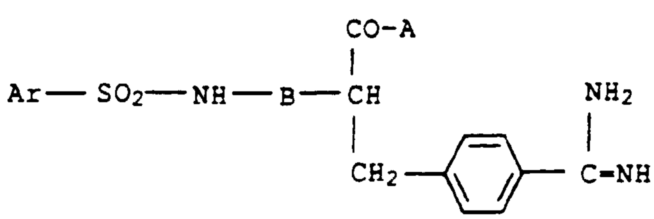

dans laquelle

A représente

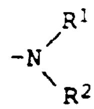

ou $-OR^3$, dans lesquels $R^1$ et $R^2$, ayant des significations identiques ou différentes, réprésentent l'hydrogène, des groupes alkyle saturés ou insaturés contenant jusqu'à 6 atomes de carbone ou des groupes aralkyle ou aryle, ou bien $R^1$ et $R^2$ forment ensemble et avec l'atome d'azote qui les relie un cycle saturé de 5 à 7 chaînons qui peut éventuellement être substitué par des groupes alkyle contenant jusqu'à 4 atomes de carbone et qui peut contenir un atome d'oxygène, les atomes d'azote et d'oxygène se trouvant dans les positions 1,2 ou 1,4, et $R^3$ représente l'hydrogène, un groupe alkyle saturé ou insaturé contenant jusqu'à 6 atomes de carbone, un groupe cycloalkyle ou un groupe aralkyle,

B représente

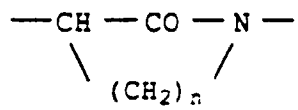

avec n valant 1,2,3, 4 ou 5
ou le groupe

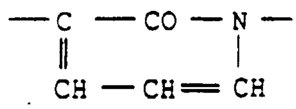

Ar représente un groupe phényle ou alpha- ou bêta-naphtyle éventuellement substitué par un ou plusieurs atomes d'halogène ou groupes nitro, amino, mono- ou bis-(alkyle en C1-C4)amino, hydroxy, alkyle en C1-C4, alcoxy en C1-C4, par un groupe méthylènedioxy ou éthylènedioxy, ou bien Ar représente un groupe pyridyle, quinoléyle ou isoquinoléyle, chacun d'eux éventuellement substitué par un ou plusieurs groupes alkyle en C1-C4 ou alcoxy en C1-C4,

et leurs sels d'acides acceptables pour l'usage pharmaceutique.

**2.** Procédé de préparation des composés de formule I selon la revendication 1, caractérisé par le fait que l'on fait réagir un composé de formule II

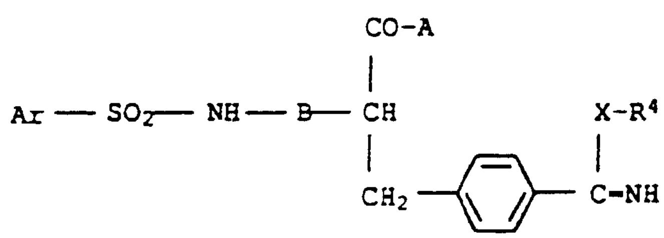

II,

dans laquelle $R^4$ représente un groupe alkyle en C1-C3 ou benzyle et X un atome d'oxygène ou de soufre, avec l'ammoniac ou un sel d'ammonium, après quoi on convertit éventuellement les composés obtenus en leurs sels d'acides acceptables pour l'usage pharmaceutique.

**3.** Composés de formule I selon la revendication 1, pour l'utilisation dans le traitement de maladies.